# EUROPEAN PATENT SPECIFICATION

(11) **EP 4 260 807 B1**
(45) Date of publication and mention of the grant of the patent: **03.09.2025**
(21) Application number: 23161572.5
(22) Date of filing: 13.03.2023
(51) Int. Cl.: A61B 6/02, A61B 6/00, A61B 6/04, A61B 6/03, A61B 6/58

(54) **X-RAY DIAGNOSIS APPARATUS AND CONTROL METHOD OF THEREOF**
RÖNTGENDIAGNOSEVORRICHTUNG UND STEUERUNGSVERFAHREN DAFÜR
APPAREIL DE DIAGNOSTIC À RAYONS X ET SON PROCÉDÉ DE COMMANDE

(30) Priority: 14.03.2022 JP 2022039585; 14.02.2023 JP 2023021145
(43) Date of publication of application: 18.10.2023
(73) Proprietor: Canon Medical Systems Corporation, Tochigi 324-0036 (JP)
(72) Inventor: IWAI, Haruki, Otawara-shi, 324-0036 (GB)
(74) Representative: Marks & Clerk LLP

(56) References cited:
- US-A1- 2010 074 483

## Description

### FIELD

The embodiments disclosed in the present specification and drawings relate to an X-ray diagnosis apparatus and a control method of thereof.

### BACKGROUND

X-ray diagnosis apparatuses are used to perform long range imaging that generate long range images by performing a plurality of shots of X-ray imaging along a direction of a body axis of a subject, collecting a plurality of X-ray images, and composing the plurality of the collected X-ray images. In such long range imaging, artifacts arise due to a Table to Object Distance (TOD) difference. That is, a size of an imaging target in X-ray imaging changes in response to an incident angle of an X-ray because a distance between the imaging target of the subject, which is the object, and a bed, which is the table, differs on the incident angle of the X-rays.

When imaging the plurality of X-ray images in long range imaging, a slit shaped imaging in which an X-ray irradiation is narrowed by a slit is performed to reduce an effect of the artifact due to the TOD difference. However, in slit shaped imaging, it is impossible to perform X-ray imaging down to the subject's feet even if an X-ray detector is moved to a lower end, since only a center portion of the X-ray detector could be used. For this reason, in long range imaging, there is a need to move the subject upward on a stool when the subject gets on the bed. Such stool is also called as a stand, for which the subject needs to get on the bed while standing on the stand to adjust a position of the subject for X-ray imaging. US 2010/074483A1 relates to an imaging system for compensating for mask frame misalignment with non-mask frames in an image sequence. The imaging system may be an X-ray imaging system. The system includes a processor for determining a compensation zoom factor for individual image frames of an image sequence in response to data indicating distance between an object of interest and a radiation detector for said individual image frames, associating individual zoom factors with corresponding individual image frames of the image sequence, storing said individual zoom factors associated with corresponding individual image frames of said image sequence in a repository, applying an individual determined zoom factor to align an associated corresponding image frame and a mask frame to provide an aligned image frame, and determining data representing an image difference frame comprising a difference between data representing said aligned image frame and a mask frame. A display image presenting said image difference frame is shown on a user interface.

### SUMMARY OF INVENTION

According to an aspect, there is provided an X-ray diagnosis apparatus according to claim 1.

The imaging control unit may adjust a range of irradiating X-ray to a subject by controlling an X-ray aperture that narrows the range where the X-ray tube irradiates X-ray to narrow a region of an end portion when X-ray imaging the X-ray image of an upper end or a lower end for the plurality of shots of X-ray imaging.

A position of the X-ray focus and the angle of irradiating X-rays to the subject may be decided in a fixed manner based on a number of shots of the X-ray imaging.

The angle of irradiating X-rays to the subject that is decided in the fixed manner may be vertically symmetrical about the position of the X-ray focus.

The imaging control unit may adjust the focus-to-detector distance in response to the angle of irradiating X-rays to the subject such that each X-ray focus of the X-ray tube is positioned at the equivalent position for the plurality of shots of X-ray imaging.

The imaging control unit may adjust a range of irradiating X-ray on a subject by controlling an X-ray aperture that narrows the range where the X-ray tube irradiates X-ray to narrow both regions of an upper end portion and a lower end portion when X-ray imaging the X-ray image of an upper end or a lower end for the plurality of shots of X-ray imaging.

The imaging control unit may adjust a range of irradiating X-ray to a subject by controlling an X-ray aperture that narrows the range where the X-ray tube irradiates X-ray to narrow a region of an end portion when X-ray imaging the X-ray image of at least one of an upper end or a lower end for the plurality of shots of X-ray imaging.

The imaging control unit may adjust a range of irradiating X-ray on a subject by controlling an X-ray aperture that narrows the range where the X-ray tube irradiates X-ray in response to a position of an upper end of long range imaging and a number of shots of X-ray imaging, to narrow an upper end portion for a first shot of X-ray imaging when X-ray imaging the X-ray image of an upper end for the plurality of shots of X-ray imaging.

The position of the X-ray focus may be set to a center portion of the range to image the subject by the plurality of shots of X-ray imaging.

The position of the X-ray focus may be set based on an inspection information about an inspection performed on the subject.

The position of the X-ray focus may be set in response to an input operation received by an input operation from an operator about setting the position of the X-ray focus.

The X-ray diagnosis apparatus may further comprise an image generation unit that generates a long range image of a subject by composing the plurality of X-ray images acquired by the plurality of shots of X-ray imaging.

The imaging control unit may control the X-ray tube such that the angle of irradiating X-rays to the subject rotates around a center of rotation located on a central axis of the X-ray detector.

The imaging control unit may control the X-ray tube such that the angle of irradiating X-rays to the subject rotates around the position of the X-ray focus of the X-ray tube.

According to a further aspect, there is provided a method according to claim 15.

The equivalent position may be an essentially equivalent position where no TOD difference arises.

The angle of irradiating X-ray to the subject that is determined in the fixed manner may be vertically nonsymmetrical about the position of X-ray focus.

The position of X-ray focus may be set to a position other than the center portion of the range to image the subject by the plurality of shots of X-ray imaging.

The center of rotation located on the central axis of the X-ray detector may be the center portion of the X-ray detector.

The center of rotation located on the central axis of the X-ray detector may be the offset position, which is the position offset from the center portion of the X-ray detector on the central axis of the X-ray detector.

### BRIEF DESCRIPTION OF THE DRAWINGS

Fig. 1 is a block diagram that describes an overall configuration of an X-ray diagnosis apparatus according to a first embodiment.
Fig. 2A is a diagram that schematically illustrates an exemplary motion of an X-ray tube holding apparatus when performing oblique incidence imaging in the X-ray diagnosis apparatus of Fig. 1.
Fig. 2B is a diagram that schematically illustrates another exemplary motion of the X-ray tube holding apparatus when performing oblique incidence imaging in the X-ray diagnosis apparatus of Fig. 1.
Fig. 3 is a diagram that illustrates a flowchart describing a content of an X-ray imaging process executed in the X-ray diagnosis apparatus of Fig. 1.
Fig. 4 is a diagram that illustrates an exemplary alignment guide screen displayed on a display comprised by the X-ray diagnosis apparatus of Fig. 1.
Fig. 5 is a diagram (fixed stroke method) that describes a range of X-ray irradiation during each shot of X-ray imaging in a state of viewing a subject from one side, for a case when performing long range imaging with three shots of X-ray imaging in the X-ray diagnosis apparatus according to the first embodiment.
Fig. 6 is a diagram that describes the range of X-ray irradiation during each shot of X-ray imaging in a state of viewing a subject from one side, for a case when performing long range imaging with two shots of X-ray imaging in the X-ray diagnosis apparatus according to the first embodiment.
Fig. 7 is a diagram that describes a position and angle of the X-ray tube holding apparatus 12 in a first shot of X-ray imaging in the state of viewing the subject from one side, for the case when performing long range imaging with three shots of X-ray imaging in the X-ray diagnosis apparatus according to the first embodiment.
Fig. 8 is a diagram that describes the position and angle of the X-ray tube holding apparatus 12 in a second shot of X-ray imaging in the state of viewing the subject from one side, for the case when performing long range imaging with three shots of X-ray imaging in the X-ray diagnosis apparatus according to the first embodiment.
Fig. 9 is a diagram that describes the position and angle of the X-ray tube holding apparatus 12 in a third shot of X-ray imaging in the state of viewing the subject from one side, for the case when performing long range imaging with three shots of X-ray imaging in the X-ray diagnosis apparatus according to the first embodiment.
Fig. 10 is a diagram (fixed stroke method) that describes the range of X-ray irradiation during each shot of X-ray imaging in the state of viewing the subject from one side, for the case when performing long range imaging with three shots of X-ray imaging in the X-ray diagnosis apparatus according to a second embodiment.
Fig. 11 is a diagram (stroke-free method) that describes the range of X-ray irradiation during each shot of X-ray imaging in the state of viewing the subject from one side, for the case when performing long range imaging with three shots of X-ray imaging in the X-ray diagnosis apparatus according to a third embodiment.
Fig. 12 is a diagram that separates the range of X-ray irradiation to describe a state where an aperture of X-ray irradiation is fully opened in the first shot of X-ray imaging and an upper side of X-ray irradiation is narrowed in the second shot of X-ray imaging.
Fig. 13 is a diagram that represents an overlap in the range of X-ray irradiation for the case where two shots of X-ray imaging shown in Fig. 12 is actually performed.
Fig. 14 is a diagram that separates the range of X-ray irradiation and describes a state where a lower side of X-ray irradiation is narrowed in the first shot of X-ray imaging and the aperture of X-ray irradiation is fully opened in the second shot of X-ray imaging.
Fig. 15 is a diagram that represents the overlap in the range of X-ray irradiation for the case where two shots of X-ray imaging shown in Fig. 14 is actually performed.
Fig. 16 is a diagram (fixed stroke method) that describes the range of X-ray irradiation during each shot of X-ray imaging in the state of viewing the subject from one side, for the case when performing long range imaging with three shots of X-ray imaging in the X-ray diagnosis apparatus according to a fourth embodiment.
Fig. 17 is a diagram (stroke-free method) that describes the range of X-ray irradiation during each shot of X-ray imaging in the state of viewing the subject from one side, for the case when performing long range imaging with three shots of X-ray imaging in the X-ray diagnosis apparatus according to a fourth embodiment.
Fig. 18 is a diagram (fixed stroke method) that describes the range of X-ray irradiation during each shot of X-ray imaging in the state of viewing the subject from one side, for the case when performing long range imaging with three shots of X-ray imaging in the X-ray diagnosis apparatus according to modified example of the first embodiment.
Fig. 19 is a diagram (stroke-free method) that describes the range of X-ray irradiation during each shot of X-ray imaging in the state of viewing the subject from one side, for the case when performing long range imaging with three shots of X-ray imaging in the X-ray diagnosis apparatus according to modified example of the third embodiment.
Fig. 20 is a diagram that illustrates an exemplary position of the X-ray focus in the X-ray diagnosis apparatus according to a modified example.

### DETAILED DESCRIPTION

With reference to the drawings below, embodiments of an X-ray diagnosis apparatus and a control method of thereof will be described. Note that, in the description below, same reference signs are given for components substantially identical in terms of configuration and function, and duplicate description will be given only when necessary.

### [First embodiment]

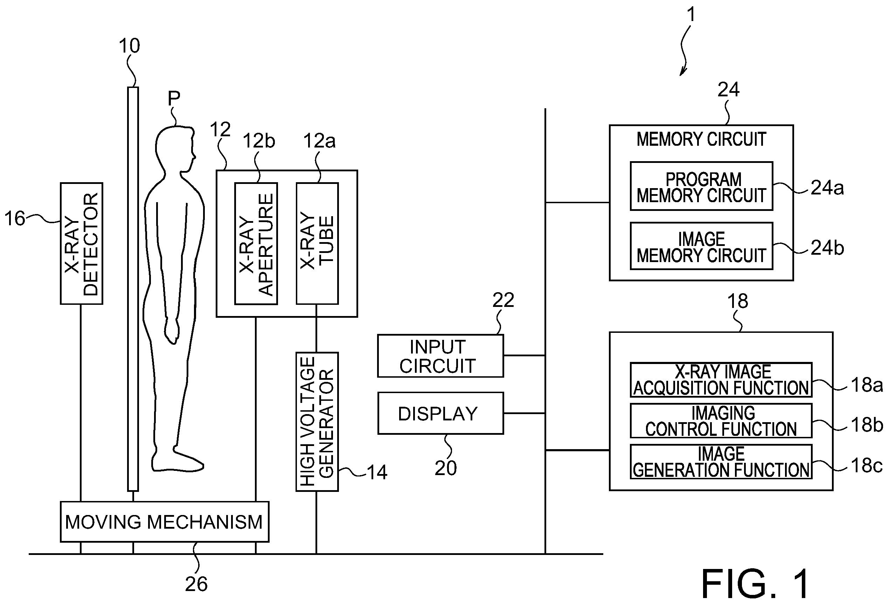

Fig. 1 is a block diagram that describes an overall configuration of the X-ray diagnosis apparatus 1 according to a first embodiment. The X-ray diagnosis apparatus 1 shown in Fig. 1 mainly comprises a bed 10, an X-ray tube holding apparatus 12, a high voltage generator 14, an X-ray detector 16, a processing circuit 18, a display 20, an input circuit 22, and a memory circuit 24. Likewise, the X-ray tube holding apparatus 12 according to the present embodiment comprises an X-ray tube 12a and an X-ray aperture 12b.

A subject P is located on the bed 10. The subject P may take an arbitrary stance such as a standing position or a face-up position. In the description below, the X-ray diagnosis apparatus 1 will be explained, for instance, with an example where the subject P is in the standing position. In the X-ray diagnosis apparatus 1 according to the present embodiment, it is possible to partially image the subject P in the standing position with one shot of X-ray imaging using the X-ray detector 16 and the X-ray tube 12a of the X-ray tube holding apparatus 12. For this reason, the X-ray detector 16 is interlocked with the X-ray tube holding apparatus 12 to be configured in a vertically movable manner and may perform X-ray imaging with a plurality of different arrangements to generate X-ray images by the plurality of different arrangements. That is, the X-ray diagnosis apparatus 1 according to the present embodiment may generate long range X-ray images by composing X-ray images by the plurality of arrangements.

X-rays are generated based on a high voltage and a filament current supplied from the high voltage generator 14 to the X-ray tube 12a of the X-ray tube holding apparatus 12 under the control of the processing circuit 18. The X-ray aperture 12b of the X-ray tube holding apparatus 12 narrow an aperture for the X-rays generated by the X-ray tube 12a to control a range of the X-rays irradiated to the subject P. That is, the irradiating range of the X-ray may be narrowed by narrowing an X-ray aperture 12b, and on the contrary, the irradiating range of the X-ray may be widened by opening the X-ray aperture 12b. A degree of narrowing of the X-ray aperture 12b, for instance, is controlled by a control signal from the processing circuit 18 based on an instruction from an operator.

The high-voltage generator 14 generates and supplies the high voltage and the filament current to the X-ray tube 12a of the X-ray tube holding apparatus 12 to generate X-ray in the X-ray tube 12a, in response to an X-ray condition based on the operating instruction from the processing circuit 18.

The X-ray detector 16, for instance, is configured by a Flat Panel Detector (FPD) having a plurality of pixels arranged in 2-dimension, where each pixel detects X-rays from the X-ray tube 12a that passed through the subject and converts the detected X-rays into an electrical signal. The electrical signal is further converted into a digital signal and output to the processing circuit 18.

The processing circuit 18 refers to a control circuit that performs an overall control of the X-ray diagnosis apparatus 1 and also an arithmetic circuit that performs various arithmetic operation. For instance, the processing circuit 18 according to the present embodiment includes an X-ray image acquisition function 18a, an imaging control function 18b, and an image generation function 18c. The X-ray image acquisition function 18a corresponds to an X-ray image acquisition unit according to the present embodiment, the imaging control function 18b corresponds to an imaging control unit according to the present embodiment, and the image generation function 18c corresponds to an image generation unit according to the present embodiment.

In the embodiment of Fig. 1, each processing function performed in the X-ray image acquisition function 18a, the imaging control function 18b, and the image generation function 18c is stored in the program memory circuit 24a of the memory circuit 24 in a form of computer executable program. The processing circuit 18 refers to a processor that realizes functions corresponding to each program by reading the program from the program memory circuit 24a of the memory circuit 24 and executing the same. In other words, the processing circuit 18 that has read each program has each function shown in the processing circuit 18 of Fig. 1. Note that in Fig. 1, it was explained as the X-ray image acquisition function 18a, the imaging control function 18b, and the image generation function 18c being realized in a single processing circuit 18, but these functions may be realized by configuring the processing circuit 18 with a plurality of independent processors and have each processor execute the program.

The display 20 displays various images or information. For instance, the display 20 displays medical images (X-ray images) generated by the processing circuit 18, or a Graphical User Interface (GUI) etc. to receive various operations from the operator. Especially, in the present embodiment, the display 20 displays a long range image, which is a long range X-ray image generated by the image generation function 18c of the processing circuit 18. In the present embodiment, the display 20, for instance, is configured by a liquid crystal display or a Cathode Ray Tube (CRT) display etc.

The input circuit 22 receives various input operations from the operator, converts the received input operations into electric signal, and outputs to the processing circuit 18. For instance, the input circuit 22 is realized by a mouse, a keyboard, a trackball, a manual switch, a foot switch, a button, or a joystick etc. Likewise, if the display 20 comprises a touch panel function, the display 20 may serve as the input circuit 22.

The memory circuit 24, for instance, is realized by a Random Access Memory (RAM), a semiconductor memory element such as a flash memory, a hard disk, or an optical disk etc. The memory circuit 24 according to the present embodiment, for instance, comprises a program memory circuit 24a and an image memory circuit 24b. As described above, each program to be executed in the processing circuit 18 etc. is stored in the program memory circuit 24a. Data related to various images are stored in the image memory circuit 24b. In the present embodiment, for instance, X-ray images generated based on X-rays detected by the X-ray detector 16 are stored in the image memory circuit 24b.

As described above, in the present embodiment, the processing circuit 18, for instance, is configured by the processor. Note that the word "processor" used in above descriptions means circuits such as, for example, a Central Processing Unit (CPU), a Graphics Processing Unit (GPU), an Application Specific Integrated Circuit (ASIC), a programmable logic device (for example, a Simple Programmable Logic Apparatus (SPLD), a Complex Programmable Logic Apparatus (CPLD), and a Field Programmable Gate Array (FPGA)). The processor executes functions by reading and executing programs stored in the memory. Note that programs may be configured to be directly integrated in the processor instead of being storing in the memory. In this case, the processor realizes functions by reading and executing programs stored in the circuit. Note that the processor is not limited to be arranged as a single processor circuit, but may be configured as a single processor by combining a plurality of independent circuits to realize functions. Furthermore, a plurality of component elements in Fig. 1 may be integrated into one processor to realize the functions.

A moving mechanism 26 refers to a mechanism that moves the bed 10, the X-ray detector 16, and the X-ray tube holding apparatus 12 in an arbitrary manner. The moving mechanism 26 performs X-ray imaging to the subject P from a plurality of imaging positions to realize long range imaging according to the present embodiment. The moving mechanism 26, for instance, is configured by a drive unit that mechanically supports the bed 10, the X-ray detector 16, and the X-ray tube holding apparatus 12 and realizes the movement of the same. The moving mechanism 26 also comprises a status detector such as a potentiometer or an encoder for each axis. A detected signal detected by the status detector is output to the processing circuit 18.

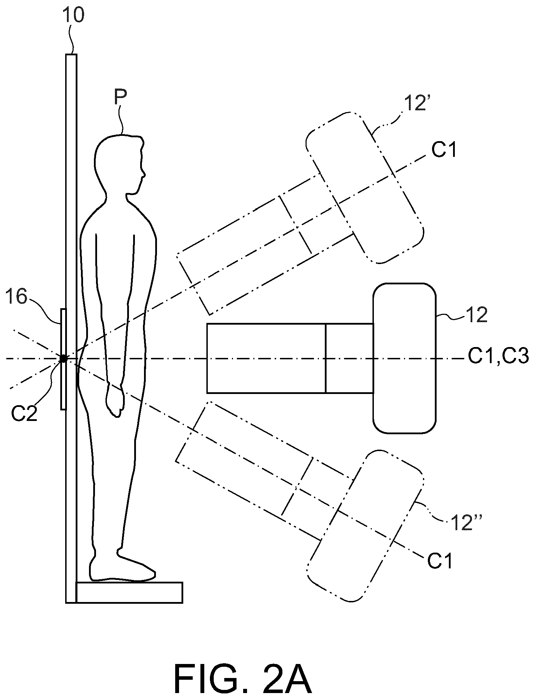

Next, a motion of the X-ray tube holding apparatus 12 in the X-ray diagnosis apparatus 1 according to the present embodiment will be explained based on Fig. 2A. Fig. 2A is a diagram that schematically illustrates the motion of the X-ray tube holding apparatus 12 when performing oblique incidence imaging in the X-ray diagnosis apparatus according to the present embodiment.

As may be seen from Fig.2A, oblique incidence imaging refers to an imaging method that obliquely irradiates X-rays to an imaging target of the subject P by tilting a central axis C1 of X-ray irradiation from the X-ray tube 12a of the X-ray tube holding apparatus 12. For this reason, X-rays are also obliquely incident into the X-ray detector 16.

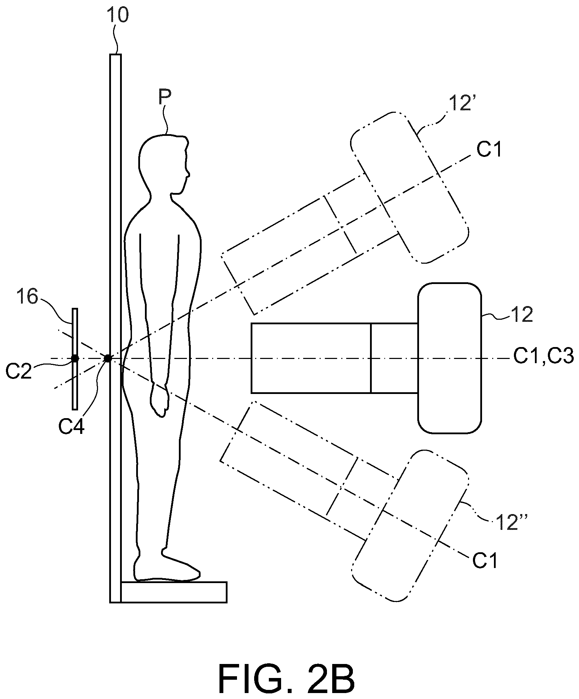

When the X-rays are vertically incident from the X-ray tube 12a of the X-ray tube holding apparatus 12 to the X-ray detector 16, the X-ray tube holding apparatus 12 is in a position of the solid line shown in Fig. 2A. For this reason, X-rays on the central axis C1 irradiated from the X-ray tube 12a of the X-ray tube holding apparatus 12 are incident to a center C2 of the X-ray detector 16 vertically. In the present embodiment, such general X-ray imaging is appropriately called as a vertical imaging to be differentiated with oblique incidence imaging. As shown in Fig. 2B when performing vertical imaging, the central axis C1 of X-ray irradiation from the X-ray tube 12a of the X-ray tube holding apparatus 12 and the central axis C3 of the X-ray detector 16 are designed to match.

On the other hand, when performing oblique incidence imaging, the X-ray tube holding apparatus 12, for instance, rotates in a clockwise direction and a counterclockwise direction in Fig. 2 around a center portion of the X-ray detector 16, which is a center of rotation located on the central axis C3 of the X-ray detector 16. The stance for which the X-ray tube holding apparatus 12 has rotated counterclockwise in Fig. 2A is indicated as 12'. That is, the X-ray tube holding apparatus 12 rotates upward around the center portion of the X-ray detector 16. For this reason, the X-rays on the central axis C1 of X-ray irradiation is also incident from obliquely above to the center C2 of the X-ray detector 16.

On the contrary, when the X-ray tube holding apparatus 12 rotates clockwise in Fig. 2A around the center portion of the X-ray detector 16, the X-ray tube holding apparatus 12 is in a stance indicated as 12". That is, the X-ray tube holding apparatus 12 rotates downward around the center portion of the X-ray detector 16, which is the center of rotation on the central axis C3 of the X-ray detector 16. For this reason, the X-ray on the central axis C1 of X-ray irradiation is also incident from obliquely below to the center C2 of the X-ray detector 16.

For this reason, in the X-ray diagnosis apparatus 1 according to the present embodiment, the X-ray tube 12a is controlled such that the angle of irradiating X-rays to the subject P from the X-ray tube 12a rotates around the center portion of the X-ray detector 16. That is, oblique incidence imaging of X-ray of various incident angles as shown in Fig. 2A is realized by adjusting the position and orientation of the X-ray tube 12a. Note that, as known from the description above, "rotate" refers to a movement that rotates around a center of a central angle with a partial rotary motion under a limitation of a specific range of the central angle.

Note that, in the X-ray diagnosis apparatus 1 according to the present embodiment described above, for instance, the X-ray tube holding apparatus 12 rotates clockwise and counterclockwise around the center portion of the X-ray detector 16, but the center of rotation of the X-ray tube holding apparatus 12 is not limited to be at the center portion of the X-ray detector 16. The center of rotation of the X-ray tube holding apparatus 12 and the center portion of the X-ray detector 16 may be located on different positions on the central axis C3 of the X-ray detector 16. Fig. 2B is a diagram that schematically illustrates another motion of the X-ray tube holding apparatus 12 when performing oblique incidence imaging in the X-ray diagnosis apparatus of Fig. 1, which corresponds to Fig. 2A. Hereinafter, parts that differ from that of Fig. 2A described above will be described.

As shown in Fig. 2B, in comparison to the X-ray diagnosis apparatus 1 shown in Fig. 2A, the X-ray detector 16 in the X-ray diagnosis apparatus 1 according to the present embodiment is horizontally moved and arranged in a direction moving away from the bed 10 and the X-ray tube holding apparatus 12. Then, the center of rotation of the X-ray tube holding apparatus 12 is at an offset position C4, which is the position offset from the center portion of the X-ray detector 16 on the central axis C3 of the X-ray detector 16.

For this reason, when X-rays are vertically irradiated from the X-ray tube 12a of the X-ray tube holding apparatus 12 to the X-ray detector 16, the X-ray tube holding apparatus 12 is at the position indicated by the solid line of Fig. 2B. For this reason, the X-rays on the central axis C1 irradiated from the X-ray tube 12a of the X-ray tube holding apparatus 12 passes the offset position C4, which is the center of rotation located on the central axis C3 of the X-ray detector 16, and is vertically incident to the center C2 of the X-ray detector 16.

On the other hand, when performing oblique incidence imaging, for instance, the X-ray tube holding apparatus 12 rotates clockwise and counterclockwise in Fig. 2B around the offset position C4, which is the center of rotation located on the central axis C3 of the X-ray detector 16. In Fig. 2B, the stance for which the X-ray tube holding apparatus 12 has rotated counterclockwise in Fig. 2B is indicated as 12'. That is, the X-ray tube holding apparatus 12 rotates upward around the offset position C4. For this reason, X-rays on the central axis C1 of X-ray irradiation passes the offset position C4 and is incident from obliquely above to the X-ray detector 16.

On the contrary, when the X-ray tube holding apparatus 12 rotates clockwise in Fig. 2B around the offset position C4, which is the center of rotation located on the central axis C3 of the X-ray detector 16, the X-ray tube holding apparatus 12 is in the stance indicated as 12". That is, the X-ray tube holding apparatus 12 rotates downward around offset position C4. For this reason, the X-ray on the central axis C1 of X-ray irradiation passes the offset position C4 and is incident from obliquely below to the X-ray detector 16.

As shown in Figs. 2A and 2B, the center of rotation of the X-ray tube holding apparatus 12 may be at the center portion of the X-ray detector 16 or the offset position C4, which is the position offset from the center portion of the X-ray detector 16 as long as the center of rotation of the X-ray tube holding apparatus 12 is on the central axis C3 of the X-ray detector 16. In the description below, details will be described with an example where the center of rotation of the X-ray tube holding apparatus 12 is at the center portion of the X-ray detector 16.

In the X-ray diagnosis apparatus 1 according to the present embodiment, the motion of the X-ray tube holding apparatus 12 to perform oblique incidence imaging is used to perform long range imaging. That is, the X-ray tube holding apparatus 12 is moved to perform the plurality of shots of X-ray imaging, but the X-ray tube holding apparatus 12 is controlled at that time such that each focus of the X-ray tube 12a is positioned at the equivalent position for each shot of X-ray imaging. Here, to be the equivalent position may refer to being physically in the equivalent position, or essentially the equivalent position where no TOD difference arises. In the description below, the X-ray tube holding apparatus 12 in the X-ray diagnosis apparatus 1 is moved and controlled such that each focus is physically at the equivalent position for each shot of X-ray imaging in the plurality of shots of X-ray imaging. That is, even when long range imaging is performed by the plurality of shots of X-ray imaging, the position of the X-ray focus of the X-ray tube 12a becomes virtually one.

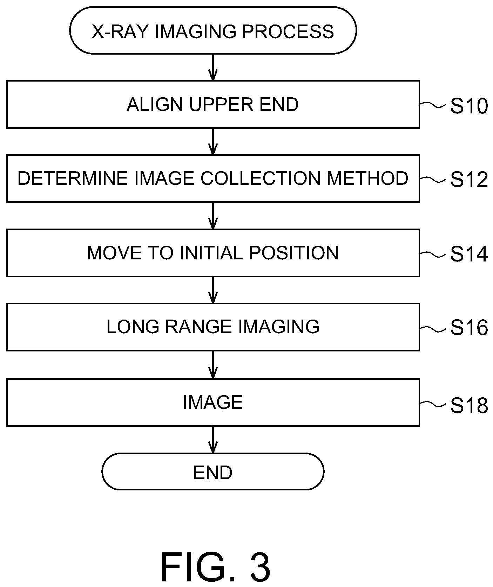

Fig. 3 is a diagram that illustrates a flowchart describing an X-ray imaging process to perform the long range imaging in the X-ray diagnosis apparatus 1 according to the present embodiment. As described above, the X-ray imaging process shown in Fig. 3 is a process realized by the processing circuit 18 reading and executing the program stored in the program memory circuit 24a of the memory circuit 24.

As shown in Fig. 3, the X-ray diagnosis apparatus 1 lets the operator to perform an upper end alignment of the long range imaging (Step S10). In the present embodiment, the imaging control function 18b in the processing circuit 18 lets the operator to perform the upper end alignment of the long range imaging. For instance, the imaging control function 18b displays an alignment guide screen on the display 20 to let the operator perform alignment.

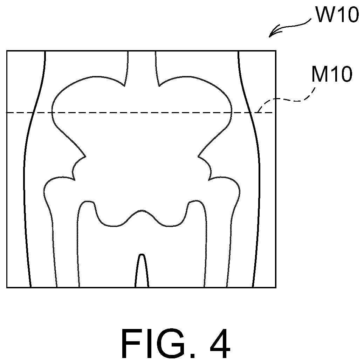

Fig. 4 is a diagram that illustrates an example of the alignment guide screen W10 displayed on the display 20 of the X-ray diagnosis apparatus 1 according to the present embodiment. As shown in Fig. 4, a position marker M10 is displayed on the alignment guide screen W10 superimposed with a fluoroscopic image of the subject P. In the present embodiment, the position marker M10 is formed by a horizontal line that indicates the upper end of the long range imaging.

To display the guide screen W10, for instance, the imaging control function 18b operates the X-ray image acquisition function 18a in the processing circuit 18, acquires the fluoroscopic image by performing fluoroscopic imaging to the subject P, and displays on the display 20. For instance, the X-ray image acquisition function 18a consecutively irradiates low dose X-ray to the subject P to acquire an image within a body of the subject P as the fluoroscopic image. The imaging control function 18b then displays the position marker M10 superimposed with the fluoroscopic image.

The fluoroscopic imaging is performed in a state of general imaging (vertical imaging) of Fig. 2A described above. That is, the X-ray tube holding apparatus 12 is at the position of the solid line of Fig. 2A. For this reason, X-rays for fluoroscopic imaging on the central axis C1 irradiated from the X-ray tube 12a of the X-ray tube holding apparatus 12 is vertically irradiated to the center C2 of the X-ray tube 16, acquiring the fluoroscopic image.

In the present embodiment, for instance, the position of the X-ray tube holding apparatus 12 and the X-ray detector 16 may be vertically moved by the operator operating the input circuit 22. The stance of the X-ray tube holding apparatus 12 at this time is in a state indicated by the solid line in Fig. 2A. That is, X-rays are vertically irradiated from the X-ray tube 12a of the X-ray tube holding apparatus 12 towards the subject P. The operator then performs fluoroscopic imaging in a vicinity of the upper end position to perform the long range imaging. Accordingly, the fluoroscopic image in the vicinity of the upper end of the long range imaging is acquired and displayed on the display 20.

In the alignment guide screen W10, the operator may operate the input circuit 22 to move the position of the position marker M10 displayed on the alignment guide screen W10. In the example of Fig. 4, the operator may move the position marker M10 in the vertical direction. That is, the operator assigns the upper end of the long range imaging and inputs to the X-ray diagnosis apparatus 1 by vertically moving the position marker M10 on the alignment guide screen W10. Accordingly, the operator may perform the upper end alignment of the long range imaging respect to the X-ray diagnosis apparatus 1.

Next, as shown in Fig. 3, the X-ray diagnosis apparatus 1 determines an image collection method to generate the long range image (Step S12). In the present embodiment, the imaging control function 18b in the processing circuit 18 determines the image collection method. Schematically, the imaging control function 18b determines a number of shots of the X-ray imaging such that the upper end position determined by the position marker M10 at Step S10 becomes the upper end of the long range imaging, narrows an upper side of the X-ray aperture 12b to image the X-ray image when imaging the X-ray image that includes the upper end, and fully opens the X-ray aperture 12b to image the X-ray images when imaging the rest of the X-ray images. Likewise, when imaging the plurality of X-ray images, the imaging control function 18b combines the motion of the X-ray tube holding apparatus 12 and the motion of the X-ray detector 16 for oblique incidence imaging described above such that the positions of the X-ray focus of the X-ray tube 12a is fixed to one position.

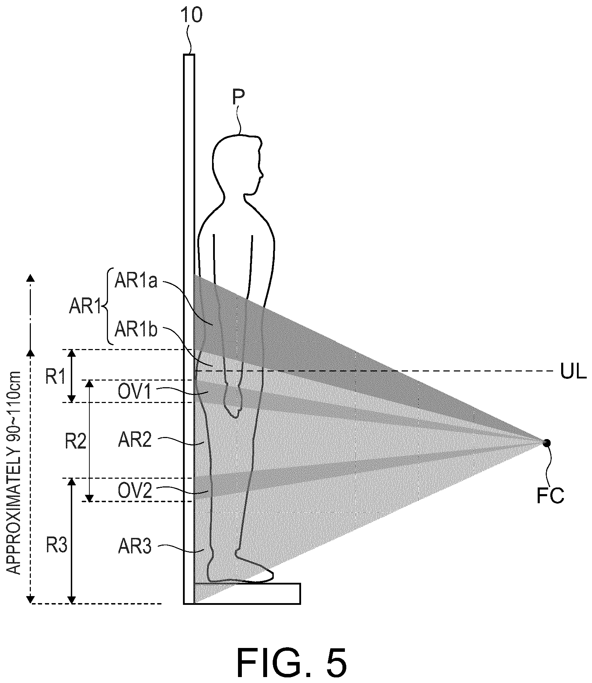

In Step S12, a procedure for which the X-ray diagnosis apparatus 1 determines the image collection method will be described using Fig. 5. Fig. 5 is a schematic diagram that describes the X-ray irradiation of the plurality of shots of X-ray imaging in a state of viewing the subject P and the bed 10 from one side. Three X-ray images are acquired by three shots of X-ray imaging in the example of Fig. 5.

More specifically, long range imaging is performed by the X-ray imaging in range AR1 of a first shot of X-ray irradiation which includes the upper end UL of the long range imaging in the imaging range, the X-ray imaging in range AR2 of a second shot of X-ray irradiation below, and the X-ray imaging of a third shot of X-ray irradiation further below. In the first shot of X-ray imaging, range AR1 of X-ray irradiation includes the upper end UL of the long range imaging designated by the operator. However, if X-rays are irradiated to all of range AR1 of X-ray irradiation in the first shot of X-ray imaging, X-rays are irradiated to above the upper end UL of the long range imaging. This results in an unnecessary exposure to the subject P.

For this reason, in the present embodiment, the upper end portion is matched to the upper end UL of the long range imaging and narrowed by the X-ray aperture 12b to adjust the range for which X-rays are irradiated to the subject P, when imaging the X-ray image of the upper end in the plurality of shots of X-ray imaging. In the example of Fig. 5, the upper end portion AR1a among range AR1 of X-ray irradiation in the first shot of X-ray imaging is narrowed by the X-ray aperture 12b and becomes the range for which X-rays are not irradiated. On the other hand, the lower end portion AR1b among range AR1 of X-ray irradiation in the first shot of X-ray imaging becomes the range which X-rays are irradiated without being narrowed by the X-ray aperture 12b. For this reason, X-ray imaging is performed on a region of the lower end portion AR1b to acquire the X-ray image of imaging range R1 in the first shot of X-ray imaging.

In the present embodiment, the upper end UL of the long range imaging is included in the region for which the X-ray image is acquired, and X-rays are irradiated, although merely, to the region above the upper end UL. This is to have the vicinity of a region of interest to be included in the X-ray imaging range because the operator may assign the vicinity of the upper end of the region of interest by the position marker M10. Note that it is also possible to design the upper end UL assigned by the operator with the position marker M10 to be a boundary of the upper end portion AR1a and the lower end portion AR1b in range AR1 of X-ray irradiation in the first shot of X-ray imaging.

Likewise, in the present embodiment, the X-ray image in imaging range R2 of the second shot of X-ray imaging is acquired by the second shot of X-ray irradiation performed on range AR2 of X-ray irradiation. Likewise, in the present embodiment, the X-ray image in imaging range R3 of the third shot of X-ray imaging is acquired by the third shot of X-ray irradiation performed on range AR3 of X-ray irradiation.

Likewise, in the present embodiment, in range AR1 of X-ray irradiation in the first shot of X-ray imaging, specifically, in the region of lower end portion AR1b, an overlapping region OV1 is included, which is the region where X-rays are irradiated overlapping with range AR2 of X-ray irradiation in the second shot of X-ray imaging. Likewise, in range AR2 of X-ray irradiation in the second shot of X-ray imaging, the overlapping region OV1 that overlaps with range AR1 of X-ray irradiation in the first shot of X-ray imaging, and an overlapping region OV2, which is the region where X-rays are irradiated overlapping with range AR3 of X-ray irradiation in the third shot of X-ray imaging, are included. Further, in range AR3 of X-ray irradiation in the third shot of X-ray imaging, the overlapping region OV2 that overlaps with range AR2 of X-ray irradiation in the second shot of X-ray imaging is included. As such, the X-ray diagnosis apparatus 1 may compose the X-ray image of imaging range R1 in the first shot of X-ray imaging, the X-ray image of imaging range R2 in the second shot of X-ray imaging, and the X-ray image of imaging range R3 in the third shot of X-ray imaging, by forming overlapping regions OV1, OV2 to generate the long range image.

Note that, in the present embodiment, range AR1 of X-ray irradiation in the first shot of X-ray imaging, range AR2 of X-ray irradiation in the second shot of X-ray imaging, and range AR3 of X-ray irradiation in the third shot of X-ray imaging may be set such that each region of the overlapping regions OV1 and OV2 are as small as possible. By such, unnecessary exposure to the subject P may be further suppressed.

In the first shot of X-ray imaging, the X-ray tube holding apparatus 12 is in the stance of oblique incidence imaging. That is, the X-ray on the central axis C1 of X-ray irradiation is incident from obliquely below to the center C2 of the X-ray detector 16. In the second shot of X-ray imaging, the X-ray tube holding apparatus 12 is in the stance of general imaging (vertical imaging). That is, the X-ray on the central axis C1 of X-ray irradiation is vertically incident to center C2 of the X-ray detector 16. In the third shot of X-ray imaging, the X-ray tube holding apparatus 12 is in the stance of oblique incidence imaging. That is, the X-ray on the central axis C1 of X-ray irradiation is incident from obliquely above to the center C2 of the X-ray detector 16.

Likewise, as may be known from Fig. 5, the position of X-ray focus FC of the X-ray tube 12a in the three shots of X-ray imaging is decided in a fixed manner regardless of the narrowing range of the first shot of X-ray imaging. That is, in the X-ray diagnosis apparatus 1 according to the present embodiment, the range that narrows the X-ray by the X-ray aperture 12b in the first shot of X-ray imaging changes by the position of the upper end UL of the long range imaging, but the position of X-ray focus FC of the X-ray tube 12a does not change.

Furthermore, the stance of the X-ray tube holding apparatus 12 in three shots of X-ray imaging is fixed regardless of the narrowing range of the first shot of X-ray imaging. That is, in the X-ray diagnosis apparatus 1 according to the present embodiment, the range that narrows the X-ray with the X-ray aperture 12b in the first shot of X-ray imaging changes by the position of the upper end UL of the long range imaging, but the stance of the X-ray tube holding apparatus 12, i.e., the angle of irradiating X-rays to the subject P does not change.

As such, once the number of shots of X-ray imaging are decided to be three, there is no need to perform complicated calculations for the position of the X-ray focus or recalculations for the stance of the X-ray tube holding apparatus 12, by fixing the position of X-ray focus FC of the X-ray tube 12a in the three shots of X-ray imaging to fix the stance of the X-ray tube holding apparatus 12, which reduces a burden of calculation when performing long range imaging in the X-ray diagnosis apparatus 1. Such aspect of the plurality of shots of X-ray imaging for long range imaging will be referred to as a fixed stroke method in the present embodiment. That is, the stroke of X-ray imaging performed three times may be understood as fixed.

With three shots of X-ray imaging as shown in Fig. 5, for instance, long range imaging may be performed with a length of approximately 90cm to 110cm. As such, when imaging a lower half of the body of the subject P as in Fig. 5, long range imaging may be performed up to a height of approximately 90cm to 110cm from a toe. When the range of long range imaging is shorter than approximately 90cm, three shots of X-ray imaging are unnecessary but two shots will suffice.

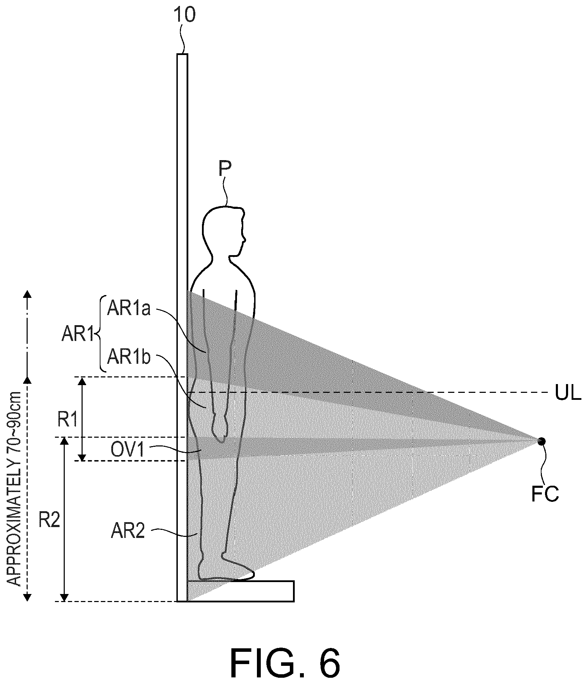

Fig. 6 is a diagram that describes an example of collecting X-ray images for long range imaging with two shots of X-ray imaging, when determining the image collection method at Step S12 in the X-ray imaging process shown in Fig. 3. Similar to Fig. 5 described above, Fig. 6 is a schematic diagram that describes the X-ray irradiation of two shots of X-ray imaging in the state of viewing the subject P and the bed 10 from one side.

As shown in Fig. 6, for instance, long range imaging for length of approximately 70cm to 90cm may be performed with two shots of X-ray imaging. In this case, long range imaging is performed with X-ray imaging on range AR1 of the first shot of X-ray irradiation that includes the upper end of long range imaging in the imaging range and the X-ray imaging on range AR2 in the second shot of X-ray irradiation below.

Similar to the description of Fig. 5, the upper end portion AR1a among range AR1 of X-ray irradiation in the first shot of X-ray imaging is narrowed by the X-ray aperture 12b and becomes the range for which X-rays are not irradiated. On the other hand, the lower end portion ARb1 among range AR1 of X-ray irradiation in the first shot of X-ray imaging becomes the range for which X-rays are irradiated, without being narrowed by the X-ray aperture 12b. That is, the length of long range imaging is adjusted by adjusting the aperture of the upper side of the X-ray aperture 12b to adjust the region of the upper end portion AR1a for which X-rays are not irradiated. Likewise, in the present embodiment, the X-ray image of imaging range R2 in the second shot of X-ray imaging is acquired by performing X-ray imaging of range AR2 of X-ray irradiation in the second shot of X-ray imaging range.

Likewise, in range AR1 of X-ray irradiation in the first shot of X-ray imaging, specifically, in the region of lower end portion AR1b, the overlapping region OV1 that overlaps with range AR2 of X-ray irradiation in the second shot of X-ray imaging is included. Likewise, in range in range AR2 of X-ray irradiation in the second shot of X-ray imaging, the overlapping region OV2 that overlaps with range AR1 of X-ray irradiation in the first shot of X-ray imaging is included. As such, the X-ray diagnosis apparatus 1 may compose the X-ray image of imaging range R1 in the first shot of X-ray imaging and the X-ray image of imaging range R2 in the second shot of X-ray imaging to generate the long range image.

Note that, in the present embodiment, range AR1 of X-ray irradiation in the first shot of X-ray imaging and range AR2 of X-ray irradiation in the second shot of X-ray imaging may be set such that each region of the overlapping regions OV1 and OV2 are as small as possible. By such, unnecessary exposure to the subject may be further suppressed.

In the first shot of X-ray imaging, the X-ray tube holding apparatus 12 is in the stance of oblique incidence imaging. That is, the X-ray on the central axis C1 of X-ray irradiation is incident from obliquely below to the center C2 of the X-ray detector 16. Also in the second shot of X-ray imaging, the X-ray tube holding apparatus 12 is in the stance of oblique incidence imaging. That is, the X-ray on the central axis C1 of X-ray irradiation is incident from obliquely above to the center C2 of the X-ray detector 16.

Likewise, as shown in Fig. 6, in two shots of X-ray imaging, once the number of shots of X-ray imaging is decided as twice, the position of X-ray focus FC of the X-ray tube 12a in each shot of X-ray imaging is fixed, as well as the stance of the X-ray tube holding apparatus 12, regardless of the position of the upper end UL of long range imaging. Accordingly, there is no need to perform complicated recalculation for the position of the X-ray focus or recalculation for the stance of the X-ray tube holding apparatus 12.

As shown in Figs. 5 and 6, the range of X-ray irradiation and the angle of irradiating X-rays decided in the fixed manner are vertically symmetric about the position of X-ray focus FC. In other words, the stroke during X-ray imaging is linearly symmetric about a line passing the position of X-ray focus FC, vertical to the bed 10.

More specifically, as may be known from comparing Figs. 5 and 6, when performing long range imaging with two shots of X-ray imaging as shown in Fig. 6, the stance of the X-ray tube holding apparatus 12 to perform the first shot of X-ray imaging and the stance of the X-ray tube holding apparatus 12 to perform the second shot of X-ray imaging becomes symmetric about the position of X-ray focus FC of the X-ray tube 12a. For this reason, the X-ray irradiation in the first shot of X-ray imaging and the X-ray irradiation in the second shot of X-ray imaging becomes symmetric about the position of X-ray focus FC of the X-ray tube 12a. On the other hand, the X-ray tube holding apparatus 12 does not perform X-ray imaging in the stance of general imaging (vertical imaging). This applies not only to the case of performing long range imaging with two shots of X-ray imaging but also to a case of performing long range imaging with an even number of shots of X-ray imaging, such as four or six shots.

On the other hand, when performing long range imaging with three shots of X-ray imaging as shown in Fig. 5, the stance of the X-ray tube holding apparatus 12 to perform the first shot of X-ray imaging and the stance of the X-ray tube holding apparatus 12 to perform the third shot of X-ray imaging becomes symmetric about the position of X-ray focus FC of the X-ray tube 12a. The stance of the X-ray tube holding apparatus 12 to perform the second shot of X-ray imaging inevitably becomes symmetric about the position of X-ray focus FC of the X-ray tube 12a, since this is the stance for general imaging (vertical imaging). This applies not only to the case of performing long range imaging with three shots of X-ray imaging but also to a case of performing long range imaging with an odd number of shots of X-ray imaging, such as five or seven shots.

Note that, generally, due to a structure of the X-ray aperture 12b, X-rays may be narrowed to only half of range AR1 of X-ray irradiation of the X-ray imaging. For this reason, it is necessary to have the length of the long range imaging that may be imaged by three shots of X-ray imaging and the length of long range imaging that may be imaged by two shots of X-ray imaging to be continuous as imaging range. For instance, the continuity of length of the long range image may be secured by adjusting the incident angle in the oblique incidence imaging or adjusting the position of X-ray focus FC of the X-ray tube 12a.

Next, as shown in Fig. 3, the X-ray diagnosis apparatus 1 moves the X-ray tube holding apparatus 12 and the X-ray detector 16 to an initial position of the long range imaging (Step S14). In the present embodiment, the imaging control function 18b in the processing circuit 18 performs the initial movement necessary to perform long range imaging. For instance, in the present embodiment, an automatic positioning button is provided as one input circuit 22, and the operator pushing the automatic positioning button becomes a trigger to perform the movement to the initial position of long range imaging.

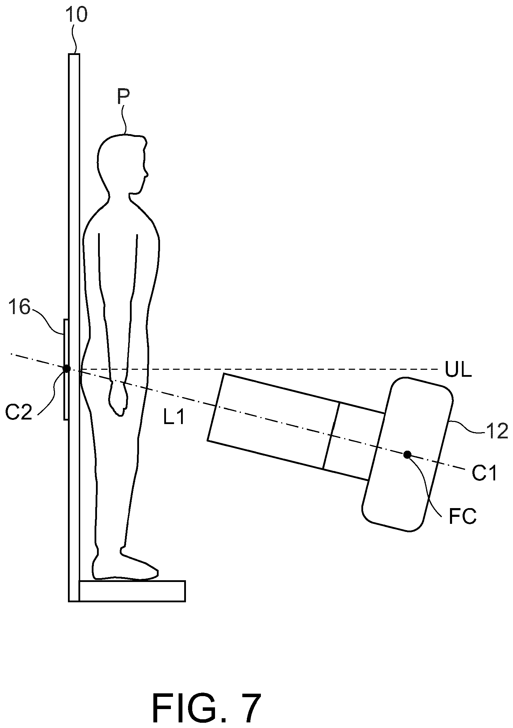

Figs. 7 to 9 are diagrams that describe motion of the X-ray tube holding apparatus 12 and the X-ray detector 16 with an example for the case when performing long range imaging with three shots of X-ray imaging. Figs. 7, 8, and 9 respectively indicate a positional relationship of the X-ray tube holding apparatus 12 and the X-ray detector 16 in the first, second, and third shot of X-ray imaging. In the description below, at Step S12 in the X-ray imaging process of Fig. 3, the motion of the X-ray diagnosis apparatus 1 will be described with the example when determining to perform long range imaging with three shots of X-ray imaging as shown in Fig. 5.

At Step S14 in the X-ray imaging process of Fig. 3, the imaging control function 18b moves the X-ray tube holding apparatus 12 and the X-ray detector 16 to the position shown in Fig. 7. That is, the imaging control function 18bmoves the X-ray tube holding apparatus 12 and the X-ray detector 16 to the position of performing the first shot of X-ray imaging for long range imaging. Note that, at Step S14, the imaging control function 18b performs the initial movement necessary to perform long range imaging for parts other than the X-ray tube holding apparatus 12 and the X-ray detector 16. For instance, the imaging control function 18b performs the initial movement of the bed 10 in response to necessity to realize the positioning of subject P assigned by the operator.

Likewise, as may be known from Fig. 7, the position and stance of the X-ray tube holding apparatus 12 to perform the first shot of X-ray imaging for long range imaging differs greatly from the position and stance of the X-ray tube holding apparatus 12 for which the upper end of long range imaging is aligned at Step S10. This is because the X-ray tube holding apparatus 12 is in the stance of general imaging (vertical imaging) shown by the solid line of Fig. 2A during alignment of the upper end at Step S10; and the X-ray tube holding apparatus 12 is in the stance of oblique incidence imaging of Fig. 2A during the movement of the X-ray tube holding apparatus 12 at Step S14.

Next, as shown in Fig. 3, the X-ray diagnosis apparatus 1 performs long range imaging (Step S16). In the present embodiment, the imaging control function 18b in the processing circuit 18 controls and executes the X-ray image acquisition function 18a to perform long range imaging. Specifically, long range imaging is realized by performing X-ray imaging shown in Figs. 7 to 9. For instance, in the present embodiment, an imaging button is provided as one input circuit 22, and after the movement to the initial position of Step S14 is complete, the operator pushing the imaging button becomes the trigger to perform long range imaging.

When executing long range imaging, the X-ray image acquisition function 18a and the imaging control function 18b in the processing circuit 18 first perform the first shot of X-ray imaging together to acquire a first X-ray image as shown in Fig. 7. As described above, X-rays are obliquely irradiated from below the subject P to the lower end portion AR1b, which is the region for X-ray imaging, among range AR1 of X-ray irradiation to acquire the X-ray image. The X-ray aperture 12b in the first shot of X-ray imaging is narrowed such that X-rays are not irradiated to the region of the upper end portion AR1a. A distance L1 from the position of X-ray focus FC of the X-ray tube 12a to the X-ray detector 16 is called as a Source-to-Image receptor distance (SID), which, in the present embodiment, is referred to as a focus-to-detector distance.

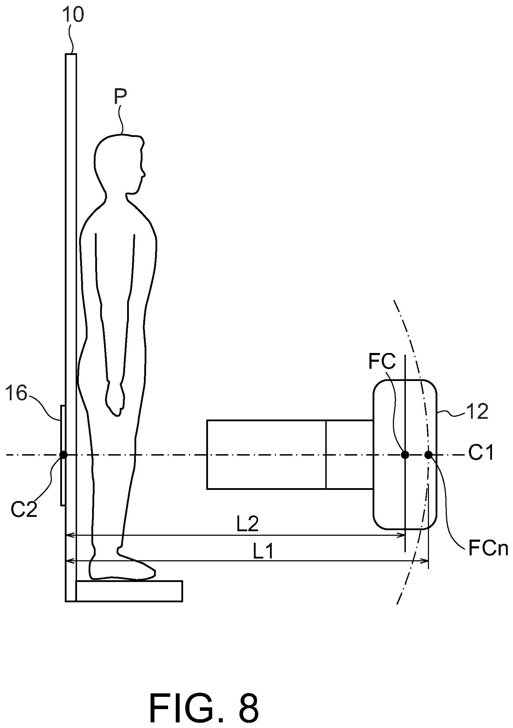

Next, as shown in Fig. 8, the X-ray image acquisition function 18a and the imaging control function 18b in the processing circuit 18 perform the second shot of X-ray imaging together to acquire a second X-ray image. As described above, in the second shot of X-ray imaging, X-rays are vertically irradiated from a front of the subject P to a whole of range AR2 of X-ray irradiation to acquire the X-ray image. That is, the X-ray that passes through the central axis C1 of X-ray irradiation is vertically irradiated to the center C2 of the X-ray detector 16. Also, the X-ray aperture 12b in the second shot of X-ray imaging is fully opened.

At this time, the imaging control function 18b adjusts the focus-to-detector distance such that the position of the X-ray focus becomes position FC as shown in Fig. 8. More specifically, as explained using Fig. 2A, the X-ray tube holding apparatus 12 of the X-ray diagnosis apparatus 1 according to the present embodiment rotates around the center portion of the X-ray detector 16. That is, the X-ray tube holding apparatus 12 performs a rotary motion such that the X-ray that passes through the central axis C1 of the X-ray tube holding apparatus 12 passes through the center C2 of the X-ray detector 16. For this reason, when the X-ray tube holding apparatus 12 performs the general rotary motion as shown in Fig. 2A, the position of X-ray focus of the X-ray tube 12a of the X-ray tube holding apparatus 12 becomes a position indicated as FCn. That is, the focus-to-detector distance becomes distance L1, which is a radius of the rotary motion, deviating the position of X-ray focus of the X-ray tube 12a from the position of X-ray focus FC shown in Fig. 7.

Thus, the imaging control function 18b in the processing circuit 18 adjusts the position of the X-ray tube holding apparatus 12 to control the rotary motion of the X-ray tube holding apparatus 12 such that the X-ray focus of the X-ray tube 12a becomes position FC. That is, the imaging control function 18b controls the focus-to-detector distance, the position of the X-ray detector 16, and the angle of irradiating X-ray to the subject P such that each X-ray focus is positioned at the equivalent position FC. Control of the focus-to-detector distance, the position of the X-ray detector 16, and the angle of irradiating X-ray to subject P is performed by the image control function 18b by obtaining a current position information about a current position of each axis of the moving mechanism 26, based on the detected signal of the of the status detector. For instance, in the example shown in Fig. 8, since the focus-to-detector distance L2 is shorter than distance L1, the image control function 18b acquires the current position of each axis of the moving mechanism 26 based on the detected signal of the status detector, and controls the X-ray tube holding apparatus 12 such that the focus-to-detector distance becomes equal to distance L2. As such, by the imaging control function 18b adjusting the focus-to-detector distance in response to the angle of irradiating X-rays to the subject P, each X-ray focus in the plurality of shots of X-ray imaging is positioned at the equivalent position FC to realize the X-ray imaging of the fixed stroke method shown in Fig. 5.

Note that, as shown in Fig. 6, when performing long range imaging with two shots of X-ray imaging, the focus-to-detector distance in the first shot of X-ray imaging and the focus-to-detector distance in the second shot of X-ray imaging are equivalent only in that the X-ray tube holding apparatus 12 performs rotary motion. For this reason, there is no need to adjust the focus-to-detector distance when imaging control function 18b in the processing circuit 18 adjusts the rotary motion of the X-ray tube holding apparatus 12 such that each X-ray focus of the X-ray tube 12a is positioned at the equivalent position FC.

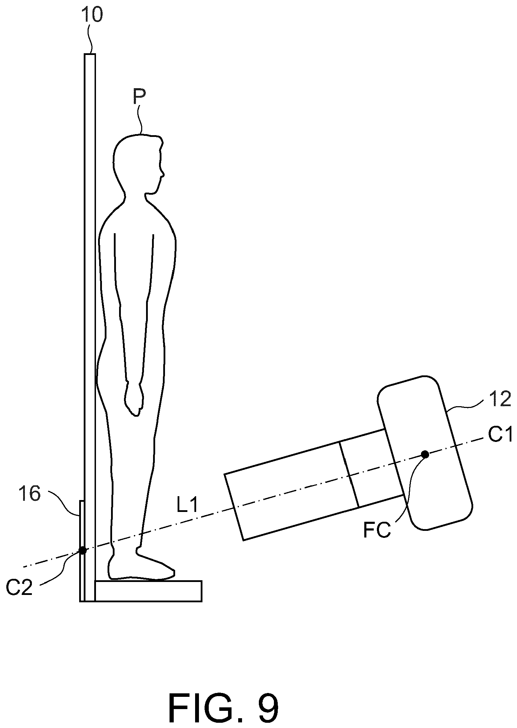

Next, as shown in Fig. 9, the X-ray image acquisition function 18a and the imaging control function 18b in the processing circuit 18 perform the third shot of X-ray imaging together to acquire a third X-ray image. As described above, in the third shot of X-ray imaging, X-rays are irradiated from obliquely above the subject P to a whole of range AR3 of X-ray irradiation to acquire the X-ray image. The focus-to-detector distance at this time is distance L1, equivalent to that of the first shot of X-ray imaging. That is, each X-ray focus FC of the X-ray tube 12a in the third shot of X-ray imaging is positioned at the equivalent position with that of the first and second shot of X-ray imaging. Likewise, the X-ray aperture 12b in the third shot of X-ray imaging is fully opened. The X-ray imaging of fixed stroke method shown in Fig. 5 ends with the third shot of X-ray imaging. That is, the long range imaging at Step S16 of the X-ray imaging process shown in Fig. 3 ends.

Note that in each of the plurality of shots of X-ray imaging, the imaging control function 18b may determine whether the focus of the X-ray tube 12a is in the equivalent position by acquiring the current position information of each axis of the moving mechanism 26. Specifically, for instance, in the example shown in Figs. 7 to 9, the image control function 18b acquires the current position information of each axis of the moving mechanism 26 based on the detected signal of the status detector in each of the first to third shots of X-ray imaging. Then, the image control function 18b may determine whether the position of the focus of the X-ray tube 12a in each of the first to third shots of X-ray imaging is in the equivalent position by calculating the focus-to-detector distance, the position of the X-ray detector 16, and the angle of irradiating X-ray to subject P based on the current position information for each of the first to third shots of X-ray imaging.

Likewise, when determining that the focus of the X-ray tube 12a is not in the equivalent position for each shot of the plurality of X-ray imaging, the image control function 18b may correct the position of the X-ray tube holding apparatus 12 so as to make the focus of the X-ray tube 12a equal, based on the calculation of the focus-to-detector distance, the position of the X-ray detector 16, and the angle of irradiating X-ray to subject P based on the current position information of each axis of the moving mechanism 26. By such, the focus of the X-ray tube 12a may be set to the equivalent position, even when sagging of the X-ray tube holding apparatus 12 occurs due to gravity and the position of the focus of the X-ray tube 12a under ideal control does not match with the position of the focus of the actual X-ray tube 12a in the X-ray diagnosis apparatus.

Next, as shown in Fig. 3, the X-ray diagnosis apparatus 1 generates the long range image of subject P by composing the plurality of X-ray images acquired at Step S16 (Step S18). In the present embodiment, the image generation function 18c in the processing circuit 18 composes the plurality of X-ray images to generate the long range image. For instance, the plurality of X-ray images imaged at Step S16 is each stored in the image memory circuit 24b of the memory circuit 24. For this reason, the image generation function 18c reads out the plurality of stored X-ray images from the image memory function 24b and generates the long range image based on the read out X-ray images.

In the example of description for Step S16, three X-ray images acquired with three shots of X-ray imaging are stored in the image memory circuit 24b. As described using Fig. 5, in the X-ray image imaged by the first shot of X-ray imaging, the region of X-ray image corresponding to the upper end portion AR1a of the first X-ray image is in a state where nothing is imaged, since X-rays are not irradiated to the upper end port AR1a. For this reason, the image generation function 18c should delete the region of X-ray image corresponding to the upper end portion AR1a of the first X-ray image by trimming.

The image generation function 18c then composes the first X-ray image for which the region corresponding to the upper end portion AR1a has been deleted, with the second and third X-ray images to generate the long range image. When composing, since overlapping regions are respectively set between the first and second X-ray images, and between the second and third X-ray images, the image generation function 18c uses the overlapping region of the X-ray images and pastes the plurality of images to generate the long range image.

Note that when the X-ray image acquired with the first shot of X-ray imaging is imaged with the X-ray aperture 12b fully opened, i.e., when only the region corresponding to the lower end portion AR1b for which X-rays are irradiated is included in the first X-ray image, the process of trimming the first X-ray image is unnecessary. That is, the image generation function 18c composes the acquired first to third X-ray images to generate the long range image without trimming the first X-ray image.

The region to trim from the first X-ray image, i.e., the region corresponding to the upper end portion AR1a for which X-rays are not irradiated, for instance, may be identified based on information related to an amount of opening of the X-ray aperture 12b. That is, information related to the amount of opening of the X-ray aperture 12b during imaging the first X-ray image is stored with the first X-ray image in the image memory circuit 24b of the memory circuit 24. Then, the image generation function 18c reads out the information related to the amount of opening of the X-ray aperture 12b from the image memory circuit 24b and identifies the region to be trimmed. Otherwise, the image generation function 18c may determine a region where there is no change of brightness as the region for which X-rays are not irradiated and identifies as the region to be trimmed.

The long range image generated at Step S18, for instance, is displayed on the display 20 or stored in the image memory circuit 24b of the memory circuit 24. By this, the X-ray imaging process according to the present embodiment shown in Fig. 3 ends.

As described above, in the X-ray diagnosis apparatus 1 according to the present embodiment, since the imaging control function 18b in the processing circuit 18 controls the focus-to-detector distance, the position of the X-ray detector 16, and the angle of irradiating X-ray to the subject P such that each X-ray focus FC of the X-ray tube 12a is positioned at the equivalent position in the plurality of images, artifacts arising from TOD difference are suppressed, and X-ray may be irradiated to the lower end of the bed 10 to perform X-ray imaging. For this reason, there is no need to have the subject stand on a stand, reducing the patient's burden. Likewise, a wide range of X-ray irradiation using the bed 10 may be secured, which widens the range of X-ray imaging.

Furthermore, since the plurality of shots of X-ray imaging was performed using the fixed stroke method shown in Figs. 5 or 6, a setting of imaging condition when performing long range imaging becomes simple compared to imaging methods that set different ranges or angles for each of the plurality of shots of X-ray imaging. For this reason, a preliminary process to perform long range imaging in the X-ray diagnosis apparatus 1 may be mitigated.

Note that in the description above, the range of irradiating X-rays to the subject P was adjusted by narrowing the region of the upper end portion AR1a when imaging the X-ray image of the upper end among the plurality of shots of X-ray imaging, but the range of irradiating X-rays to the subject P may be adjusted by narrowing the region of the lower end portion AR1b when imaging the X-ray image below. That is, in the X-ray diagnosis apparatus 1 according to the present embodiment, the imaging control function 18b may adjust the range of irradiating X-rays to the subject P by controlling the X-ray aperture 12b that narrows the range where the X-ray tube irradiates X-ray to narrows the region of an end portion when imaging the X-ray image of the upper end or the lower end in the plurality of shots of X-ray imaging.

Likewise, in the description above, the X-ray image of the upper end was first imaged and X-ray images were imaged in a downward order, but the order of imaging may be arbitrary. For instance, X-ray images may be imaged in order of bottom to top, or X-ray images of arbitrary positions may be imaged in no particular order.

### [Second Embodiment]

In the X-ray diagnosis apparatus 1 according to the first embodiment described above, the region of the upper end portion AR1a when imaging the X-ray image of the upper end was narrowed, or the region of the lower end portion AR1b when imaging the X-ray image of the lower end was narrowed, but in the X-ray diagnosis apparatus 1 according to the second embodiment, the range of X-ray irradiation is adjusted by narrowing the both the upper end portion and the lower end portion when imaging the X-ray image of the upper end or the lower end. Hereinafter, parts that differ from that of the first embodiment described above will be described.

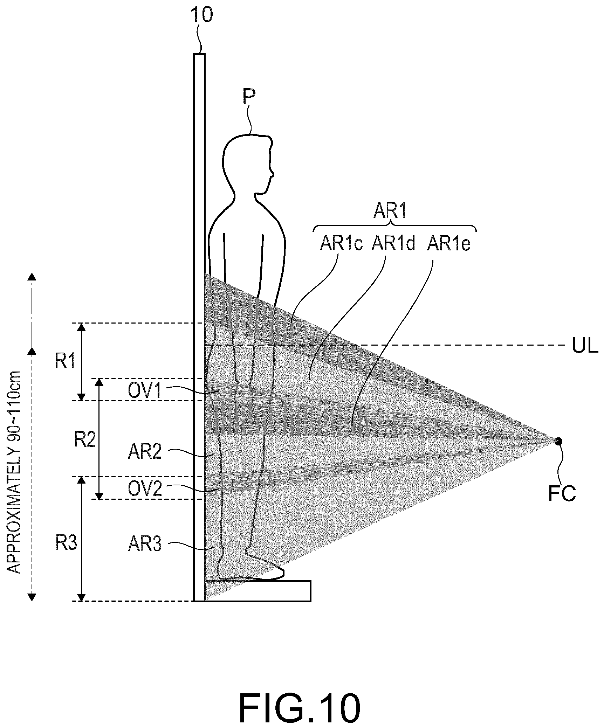

Fig. 10 is a diagram that describes an example of narrowing the upper end portion AR1c and the lower end portion AR1e among range AR1 of X-ray irradiation for X-ray imaging to irradiate X-ray to a middle portion AR1d when imaging the X-ray image of the upper end, which corresponds to Fig. 5 of the first embodiment described above. In the example of Fig. 10, the range of X-ray imaging for long range imaging is adjusted in the first shot among the plurality of shots of X-ray imaging. A narrowing amount of the upper end portion AR1c and the lower end portion AR1e may be equal or different. To make the narrowing amount of the upper end portion AR1c and the lower end portion AR1e equal, an upper wing and a lower wing of the X-ray aperture 12b are narrowed in a vertically symmetric manner. In the present embodiment, such manner of narrowing will be referred to as a symmetric control narrowing.

In such symmetric control narrowing, the middle portion AR1d includes the upper end UL of the long range imaging. In the present embodiment, the long range image is generated by composing the X-ray image of the middle portion AR1d, which is the range of first shot of X-ray irradiation, the X-ray image of range AR2 of second shot of X-ray irradiation, and the X-ray image of range AR3 of third shot of X-ray irradiation. That is, the X-ray diagnosis apparatus 1 generates the long range image by composing the X-ray image in imaging range R1 acquired by performing the first shot of X-ray imaging in the middle portion AR1d, the X-ray image in imaging range R2 acquired by performing the second shot of X-ray imaging, and the X-ray image in imaging range R3 acquired by performing the third shot of X-ray imaging. For this reason, the overlapping region OV1, which is the region where the middle portion AR1d, which is the range of first shot of X-ray irradiation, and range AR2 of second shot of X-ray irradiation overlaps is formed; and overlapping region OV2, which is the region where range AR2 of second shot of X-ray irradiation and range AR3 of third shot of X-ray irradiation overlaps is formed, to generate X-ray images of each imaging range as shown in Fig. 10.

On the other hand, when the narrowing amounts of the upper end portion AR1c and the lower end portion AR1e are not equal, for instance, in the first shot of X-ray imaging, the narrowing amount of the upper wing of the X-ray aperture 12b may be adjusted in response to the upper end UL of long range imaging and the narrowing amount of the lower wing may be fixed. In this case, the upper wing of the X-ray aperture 12b may be narrowed in the range which the upper end UL of long range imaging is included in the middle portion AR1d. In the present embodiment, such manner of narrowing will be referred to as a nonsymmetrical control narrowing.

In the second embodiment, the imaging control function 18b in the processing circuit 18 determines the image collection method to perform long range imaging to the upper end UL assigned by the operator, by the symmetrical control narrowing or the nonsymmetrical control narrowing shown in Fig. 10 at Step S12 in the X-ray imaging process shown in Fig. 3. Then, at Step S16, the imaging control function 18b in the processing circuit 18 performs long range imaging according to the determined image collection method. Aspects otherwise are equal to that of the X-ray diagnosis apparatus 1 according to the first embodiment described above.

As described above, in the X-ray diagnosis apparatus 1 according to the present embodiment, the range of X-ray irradiation when imaging the X-ray image of the upper end was adjusted by symmetrical control narrowing or nonsymmetrical control narrowing, and the imaging control function 18b in the processing circuit 18 controlled the focus-to-detector distance, the position of the X-ray detector 16, and the angle of irradiating X-ray to the subject P such that each X-ray focus FC of the X-ray tube 12a is positioned at the equivalent position in the plurality of shots of X-ray imaging. For this reason, in such manner of narrowing the range of X-ray irradiation, artifacts arising from TOD difference are suppressed, and X-ray imaging may be performed by irradiating X-rays to the lower end of the bed 10.

Note that, even in the present embodiment, the range of irradiating X-rays to the subject P may be adjusted by narrowing both the upper end AR1c and the lower end AR1e of the range of X-ray irradiation when imaging the X-ray image of the lower end among the plurality of shots of X-ray imaging, similar to the first embodiment described above. That is, in the X-ray diagnosis apparatus 1 according to the present embodiment, the range of irradiating X-rays to the subject P may be adjusted by the imaging control function 18b controlling the X-ray aperture 12b that narrows the range of X-ray irradiated by the X-ray tube 12a to narrow the region of both the upper end portion and the lower end portion when X-ray imaging the X-ray image of the upper end or the lower end among the plurality of shots of X-ray imaging.

Likewise, in the description of Fig. 10 described above, the X-ray image of the upper end was first imaged and X-ray images were consequently imaged in a downward order, but the order of imaging may be arbitrary. For instance, X-ray images may be imaged in order of bottom to top, or X-ray images of arbitrary positions may be imaged in no particular order.

### [Third Embodiment]

In the X-ray diagnosis apparatus 1 according to the first embodiment and the second embodiment described above, the position of X-ray imaging and the angle of irradiating X-rays to the subject was decided in the fixed manner in response to the number of shots of X-ray imaging as the fixed stroke method. That is, the position of X-ray focus FC was decided in the fixed manner regardless of the length of long range imaging, in response to the number of shots of X-ray. However, the position of X-ray focus FC may be set to the middle portion of the long range imaging, and the position of X-ray imaging and the angle of irradiating X-rays may be adjusted in response to the position of X-ray focus FC that is set. Such imaging method will be referred to as a stroke-free method in the present embodiment. Hereinafter, parts that differ from that of the first embodiment and the second embodiment above will be described, regarding the configuration and operation of the X-ray diagnosis apparatus 1 adopting the stroke-free method.

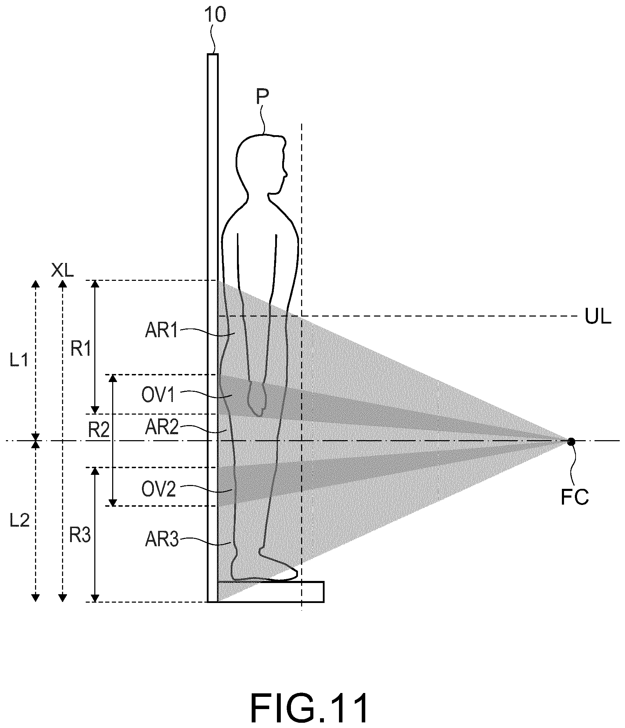

Fig. 11 is a diagram that illustrates an exemplary range of X-ray irradiation when performing long range imaging with three shots of X-ray imaging with stroke-free method. With stroke-free method, the position of X-ray focus FC is virtually set at the center portion of the range where subject P will be imaged by the plurality of shots of X-ray imaging. That is, a height of position of X-ray focus FC changes in response to the length of the long range image. In the example of Fig. 11, the position of X-ray focus FC is virtually set at the center portion in the second shot of X-ray imaging since the long range imaging is performed by three shots of X-ray imaging. That is, each X-ray focus FC is set to a height such that the upper length L1 and the lower length L2 of length XL of long range imaging are equivalent.

The number of necessary shots of X-ray imaging is determined by length XL of the X-ray imaging. That is, at Step S12 of the X-ray imaging process described above, the number of shots is determined based on the upper end UL of long range imaging designated by the operator when the imaging control function 18b in the processing circuit 18 determines the image collection method. Specifically, the imaging range when performing X-ray imaging in the state of fully opening the aperture of X-ray aperture 12b becomes the longest imaging length. For instance, even if two shots of X-ray imaging were performed while fully opening the X-ray aperture 12b, the third shot of X-ray imaging becomes necessary if the imaged length is shorter than the imaging length that includes the upper end UL designated by the operator.

When the number of shots to be imaged is determined, the imaging control function 18b in the processing circuit 18 adjusts the X-ray aperture 12b in each shot of X-ray imaging to adjust the range of X-ray irradiation. At this time, the imaging control function 18b adjusts the position of the X-ray tube 12a and the irradiation angle of X-ray such that the position of each X-ray focus FC is maintained in the equivalent position in the plurality of shots of X-ray imaging.

In the example shown in Fig. 11, the X-ray image in imaging range R1 of the first shot of X-ray imaging is acquired by performing the first shot of X-ray imaging in range AR1 of X-ray irradiation. Likewise, in the example shown in Fig. 11, the X-ray image in imaging range R2 of the second shot of X-ray imaging is acquired by performing the second shot of X-ray imaging in range AR2 of X-ray irradiation. Furthermore, in the present embodiment, the X-ray image in imaging range R3 of the third shot of X-ray imaging is acquired by performing the third shot of X-ray imaging in range AR3 of X-ray irradiation.

Likewise, the range of X-ray irradiation in the plurality of shots of X-ray imaging requires overlapping regions to compose the long range image. For instance, when the long range imaging is performed with three shots of X-ray imaging, the overlapping region OV1 of range AR1 of X-ray irradiation of the first shot of X-ray imaging and range AR2 of X-ray irradiation of the second shot of X-ray irradiation is formed, and the overlapping region OV2 of range AR2 of X-ray irradiation of the second shot of X-ray imaging and range AR3 of X-ray irradiation of the third shot of X-ray imaging is formed. The overlapping regions are better to be as small as possible in order to evade unnecessary exposure. For this reason, the imaging control function 18b determines the focus-to-detector distance, the position of the X-ray detector 16, and the angle of irradiating X-ray to the subject P so as to meet these conditions.

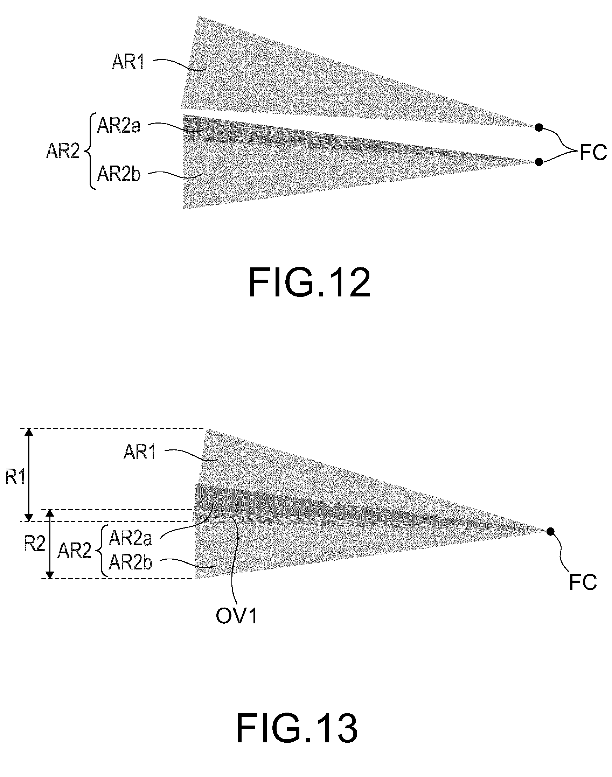

Fig. 12 is a diagram that separates and illustrates range AR1 of the first shot of X-ray irradiation and range AR2 of the second shot of X-ray irradiation, which indicates an example of narrowing the upper end portion AR2a of range AR2 of the second shot of X-ray irradiation with the X-ray aperture 12b. That is, the X-ray aperture 12b is fully opened in range AR1 of the first shot of X-ray irradiation. Then, in range AR2 of the second shot of X-ray irradiation, the upper side is narrowed by the X-ray aperture 12b, irradiating X-rays to the lower end portion AR2b of range AR2 of X-ray irradiation. By such, the region in which X-rays are overlapped and irradiated is made as small as possible. When X-rays are actually irradiated to perform X-ray imaging, the range of X-ray irradiation is as shown in Fig. 13. That is, the overlapping region OV1 in which X-ray irradiation overlaps is formed to compose the X-ray image in imaging range R2 in the second shot of X-ray imaging, but the area of the same is suppressed to be as small as possible.

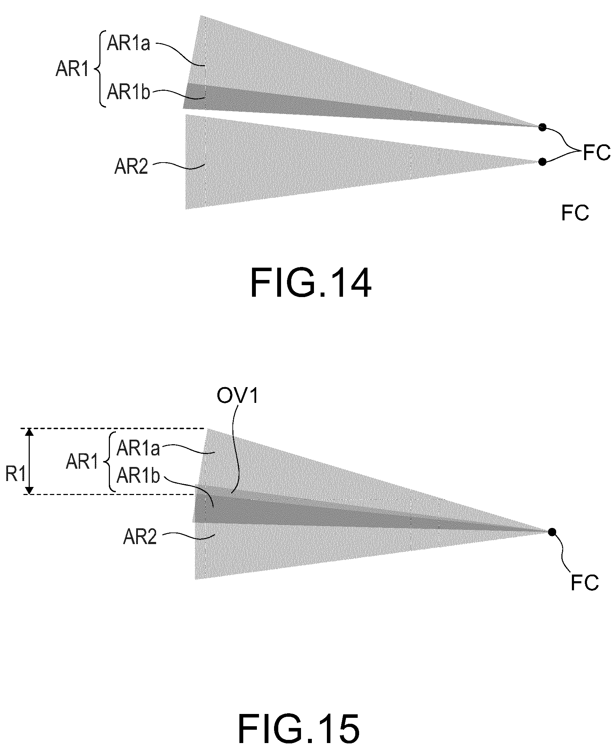

Fig. 14 is a diagram that separates and illustrates range AR1 of the first shot of X-ray irradiation and range AR2 of the second shot of X-ray irradiation, which indicates an example of narrowing the lower end portion AR1b of range AR1 of the first shot of X-ray irradiation with the X-ray aperture 12b. That is, in range AR1 of the first shot of X-ray irradiation, the lower side is narrowed by the X-ray aperture 12b, and X-rays are irradiated to the upper end portion AR1a. In range AR2 of the second shot of X-ray irradiation, the X-ray aperture 12b is fully open. Then, by such, the region where X-rays are overlapped and irradiated is made as small as possible. When X-rays are actually irradiated to perform X-ray imaging, the range of X-ray irradiation is as shown in Fig. 15. That is, the overlapping region OV1 in which X-ray irradiation overlaps is formed to compose the X-ray image in imaging range R1 of the first shot of X-ray imaging and the X-ray image in imaging range R2 of the second shot of X-ray imaging, but the area of the same is suppressed to be as small as possible.

Note that, in the stroke-free method, both the upper end portion and the lower end portion of the range of X-ray irradiation may be narrowed by the X-ray aperture 12b. That is, the imaging control function 18b in the processing circuit 18 may adjust the range of irradiating X-rays to the subject P by controlling the X-ray aperture 12b that narrows the range where the X-ray tube 12a irradiates X-ray, to narrow the region of the end portion when imaging the X-ray image of at least one of the upper end and the lower end in the plurality of shots of X-ray imaging.

Likewise, in the examples shown in Figs. 12 to 15, the description focused on range AR1 of the first shot of X-ray irradiation and range AR2 of the second shots of X-ray irradiation, but the region where X-rays are overlapped and irradiated may be made as small as possible as well as for the overlapping region OV2 of range AR2 of the second shot of X-ray irradiation and range AR3 of the third shot of X-ray irradiation, similar to the overlapping region OV1 of range AR1 of the first shot of X-ray irradiation and range AR2 of the second shot of X-ray irradiation, by narrowing the lower end portion of range AR2 of the second shot of X-ray irradiation with the X-ray aperture 12b and/or narrowing the upper end portion of range AR3 of the third shot of X-ray irradiation with the X-ray aperture 12b.

At Step S18 of the X-ray imaging process, when composing the plurality of X-ray images imaged at Step S16, the region where X-rays were not irradiated, being narrowed by the X-ray aperture 12b, is trimmed during the image composition and not used. By such, the long range image of the subject P below the upper end UL of the long range imaging designated by the operator at Step S10 may be acquired.

As described above, according to the X-ray diagnosis apparatus 1 of the present embodiment, the imaging control function 18b in the processing circuit 18 controls the focus-to-detector distance, the position of the X-ray detector 16, and the angle of irradiating X-ray to the subject P along with determining the range of X-ray irradiation by the stroke-free method such that each X-ray focus FC of the X-ray tube 12a is positioned at the equivalent position in the plurality of shots of X-ray imaging. For this reason, artifacts arising from the TOD difference are suppressed, and X-ray imaging may be performed to the lower end of the bed 10 to perform X-ray imaging even in stroke-free methods.

Note that the X-ray image of the upper end was first imaged and X-ray images were consequently imaged in a downward order, but the order of imaging may be arbitrary. For instance, X-ray images may be imaged in order of bottom to top, or X-ray images of arbitrary positions may be imaged in no particular order.

### [Fourth Embodiment]

In the X-ray diagnosis apparatus 1 according to the first to third embodiments described above, the X-ray tube 12b and the X-ray tube holding apparatus 12 performed long range image by performing rotary motion with the center of rotation at the center C2 of the X-ray detector 16 such that the angle of irradiating X-rays to the subject P rotates around center C2 of the X-ray detector 16. However, long range imaging may be performed by the X-ray tube 12a performing a swinging motion around the position of X-ray focus of the X-ray tube 12a such that the angle of irradiating X-rays to the subject P rotates around the focal position of the X-ray tube 12a. Hereinafter, the X-ray diagnosis apparatus 1 where the X-ray tube 12a performs swinging motion will be described as the X-ray diagnosis apparatus 1 according to a fourth embodiment, as well as parts that differ from that of the first to third embodiments.

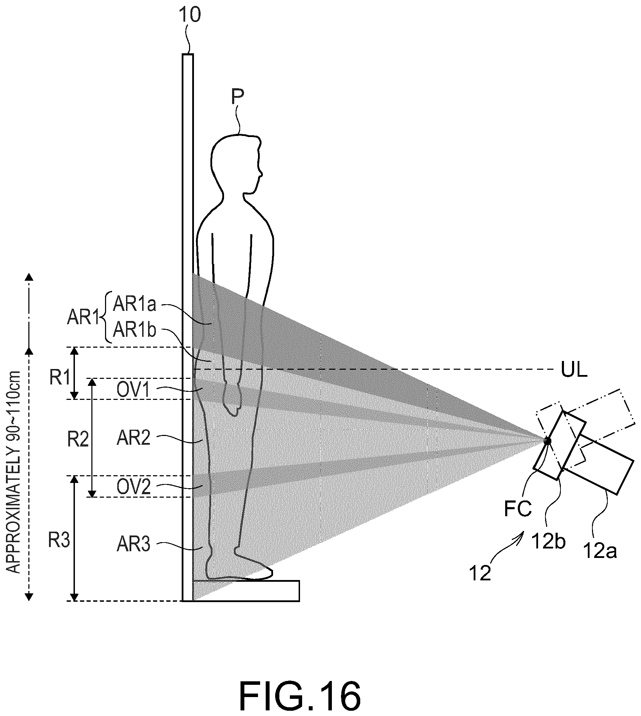

Fig. 16 is a diagram indicating the range of X-ray irradiation in the plurality of shots of X-ray imaging that describes the swinging motion of the X-ray tube 12a in the X-ray diagnosis apparatus 1 according to the present embodiment, which corresponds to Fig. 5 according to the first embodiment described above. As shown in Fig. 16, in the present embodiment, the swinging motion of the X-ray tube 12a is realized by the X-ray tube holding apparatus 12 which holds the X-ray tube 12a performing swinging motion.

That is, the X-ray tube holding apparatus 12 rotates in a state of holding the X-ray tube 12a and the X-ray aperture 12b such that the X-ray tube 12a rotates around the position of X-ray focus FC of the X-ray tube 12a. In other words, the imaging control function 18b in the processing circuit 18 rotates the X-ray tube holding apparatus 12 such that the angle of irradiating X-ray to the subject P rotates around the position of X-ray focus FC of the X-ray tube 12a. For this reason, each X-ray focus is inevitably positioned at the equivalent position FC in the plurality of shots of X-ray imaging. Regarding the focus-to-detector distance, which is the distance from the position of X-ray focus FC of the X-ray tube 12a to the X-ray detector 16, no special adjustment is necessary in the plurality of shots of X-ray imaging.

Similar to that of Fig. 5 described above, in the example of Fig. 16, the upper end portion AR1a is narrowed by the X-ray aperture 12b in the first shot of X-ray imaging, and X-rays are not irradiated. On the other hand, X-ray is irradiated to the lower end portion AR1b without being narrowed by the X-ray aperture 12b. That is, X-ray is irradiated for the region of bottom end portion AR1b and the X-ray image of imaging range R1 in the first shot of X-ray imaging is acquired. Likewise, in the second and third shots of X-ray imaging, the X-ray aperture 12b is fully opened, and the range of X-ray irradiation becomes range AR2 and range AR3, respectively. That is, the X-ray images of imaging range R2 and imaging range R3 are acquired by performing the second and third shot of X-ray imaging.

Likewise, similar to the example shown in Fig. 5 described above, in the example shown in Fig. 16, the overlapping range OV1, which is the region where the lower end portion AR1b that is the range of X-ray irradiation for the first shot and range AR2 of the second shot of X-ray irradiation overlap is formed; and the overlapping region OV2, which is the region where range AR2 of the second shot of X-ray irradiation and range AR3 of the third shot of X-ray irradiation overlap is formed, to compose the X-ray images of each imaging range. As such, long range imaging may be realized by the plurality of shots of X-ray images of the fixed stroke method such as in the first and second embodiments described above, even for the X-ray diagnosis apparatus 1 comprising the X-ray tube 12a performing swinging motion.

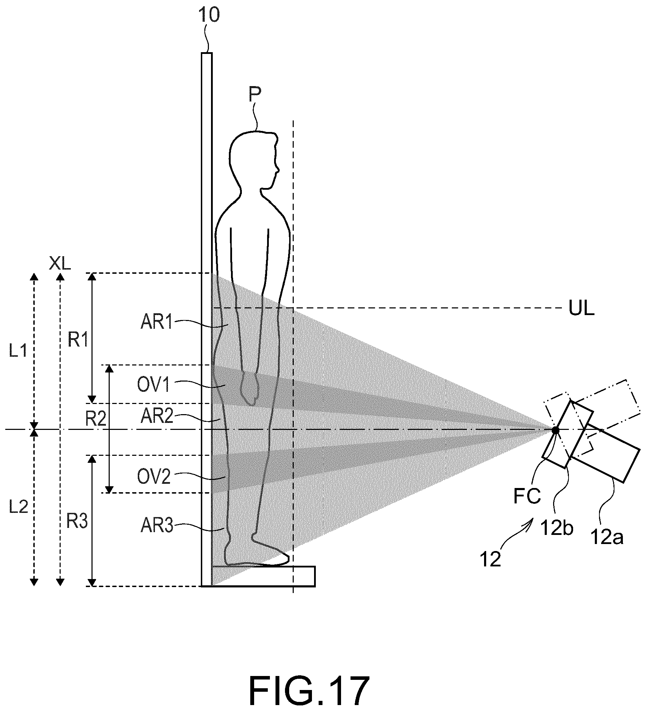

Fig. 17 is a diagram indicating X-ray irradiation in the plurality of shots of X-ray imaging that describes the swinging motion of the X-ray tube 12a in the X-ray diagnosis apparatus 1 according to the present embodiment, which corresponds to Fig. 11 according to the third embodiment described above. In the example of Fig. 17, the position of X-ray focus FC is set to the center portion of the range to be imaged by long range imaging.

That is, the X-ray focus FC of the X-ray tube 12a is set to a height where the length L1 of the upper side and the length L2 of the lower side of the long range imaging becomes equivalent heights, and the X-ray tube holding apparatus 12 rotates in the state of holding the X-ray tube 12a and the X-ray aperture 12b such that the X-ray tube 12a rotates around the position of X-ray focus FC. In other words, the imaging control function 18b in the processing circuit 18 rotates the X-ray tube 12a such that the angle of irradiating X-rays to the subject P rotates around the position of X-ray focus FC of the X-ray tube 12a. For this reason, each X-ray focus FC is inevitably positioned at the equivalent position in the plurality of shots of X-ray imaging.

Similar to that of the third embodiment described above, in the example of Fig. 17, the imaging control function 18b in the processing circuit 18 adjusts the range of irradiating X-rays to the subject P by controlling the X-ray aperture 12b that narrows the range where the X-ray tube 12a irradiates X-rays to narrow the region of the end portion when imaging the X-ray image of at least one of the upper end and the lower end in the plurality of shots of X-ray imaging. That is, in the example shown in Fig. 17, the X-ray images in imaging range R1, R2, and R3 in the first, second, and third shot of X-ray imaging is acquired by respectively performing the first shot of X-ray imaging in range AR1 of X-ray imaging, the second shot of X-ray imaging in range AR2 of X-ray imaging, and the third shot of X-ray imaging in range AR3 of X-ray imaging.

Similar to that of the third embodiment described above, in the example shown in Fig. 17, the overlapping range OV1, which is the region where range AR1 of the first shot of X-ray irradiation and range AR2 of the second shot of X-ray irradiation overlap is formed; and the overlapping region OV2, which is the region where range AR2 of the second shot of X-ray irradiation and range AR3 of the third shot of X-ray irradiation overlap is formed. As such, long range imaging may be realized by the plurality of shots of X-ray images of the stroke-free method such as in the third embodiment described above even for the X-ray diagnosis apparatus 1 comprising the X-ray tube 12a performing swinging motion.

As described above, according to the X-ray diagnosis apparatus 1 of the present embodiment, the imaging control function 18b in the processing circuit 18 controls the X-ray tube holding apparatus 12 that comprises the X-ray tube 12a such that the angle of irradiating X-rays to the subject P rotates around the focal position of the X-ray tube 12a to image the plurality of X-ray images, and generates the long range image.

### [Modified Example of First, Second, and Fourth Embodiments]

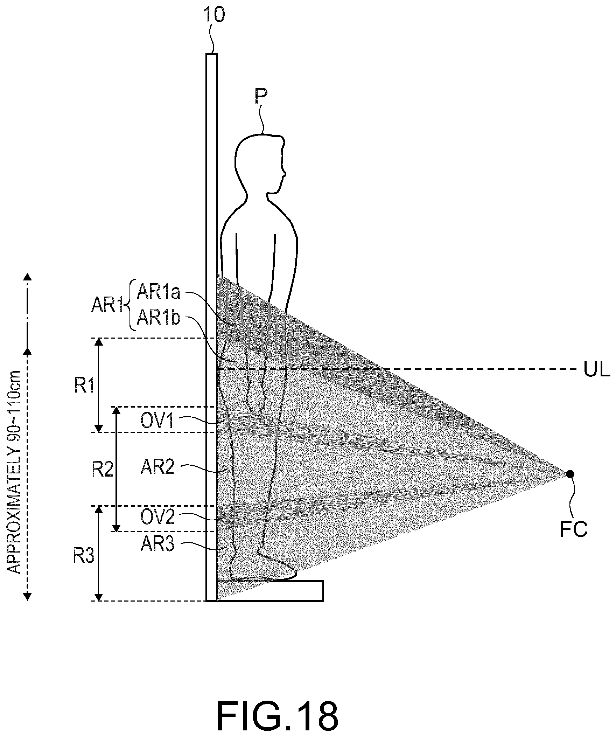

Likewise, in the first, second, and fourth embodiments, the range of X-ray irradiation and the angle of irradiating X-rays decided in the fixed manner was vertically symmetrical about the position of X-ray focus FC when performing X-ray imaging with the fixed stroke method, but the range of X-ray irradiation and the angle of irradiating X-rays decided in the fixed manner may be nonsymmetrical about the position of X-ray focus FC. For instance, as shown in Fig. 18, when performing long range imaging with three shots of X-ray imaging, the range of X-ray irradiation and the angle of irradiating X-rays decided in the fixed manner in the first shot of X-ray imaging, and the range of X-ray irradiation and the angle of irradiating X-rays decided in the fixed manner in the third shot of X-ray imaging may be nonsymmetrical about the position of X-ray focus FC. For this reason, the stance of the X-ray tube holding apparatus 12 in the first shot of X-ray imaging and the stance of the X-ray tube holding apparatus 12 in the third shot of X-ray imaging may be nonsymmetrical about the position of X-ray focus FC of the X-ray tube 12a. Also, the X-ray diagnosis apparatus 1 may adjust the focus-to-detector distance in response to the angle of irradiating X-rays to the subject P such that each X-ray focus in the plurality of shots of X-ray imaging is positioned at the equivalent position.

Note that, in the first, second, and fourth embodiments, similar to when performing long range imaging with three shots of X-ray imaging, when performing long range imaging with two shots of X-ray imaging, the stance of the X-ray tube holding apparatus 12 in the first shot of X-ray imaging and the stance of the X-ray tube holding apparatus 12 in the second shot of X-ray imaging may be nonsymmetrical about the position of X-ray focus FC of the X-ray tube 12a.

### [Modified Example of Third and Fourth Embodiments]

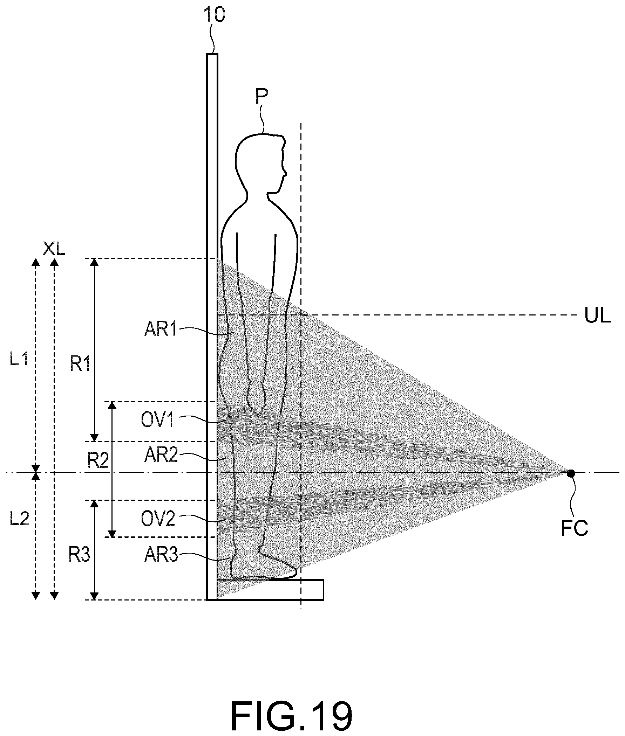

In the third and fourth embodiments described above, the position of X-ray focus FC was set to the center portion of long range imaging when performing X-ray imaging with the stroke-free method, but the position of X-ray focus FC may be set to a position other than the center portion of long range imaging. For instance, as shown in Fig. 19, the position of X-ray focus FC may be set such that length L1 of the upper side and length L2 of the lower side for length XL of long range imaging become different heights. That is, the height of the position of X-ray focus FC changes in response to the height of long range imaging, length L1 of the upper side, and length L2 of the lower side. Also, the X-ray diagnosis apparatus may adjust the position of performing X-ray imaging and the angle of irradiating X-rays in response to the number of shots of imaging and the position of X-ray focus FC that is set, such that each X-ray focus FC in the plurality of shots of X-ray imaging is positioned at the equivalent position.

### [Modified Example for Each Embodiment in Common]

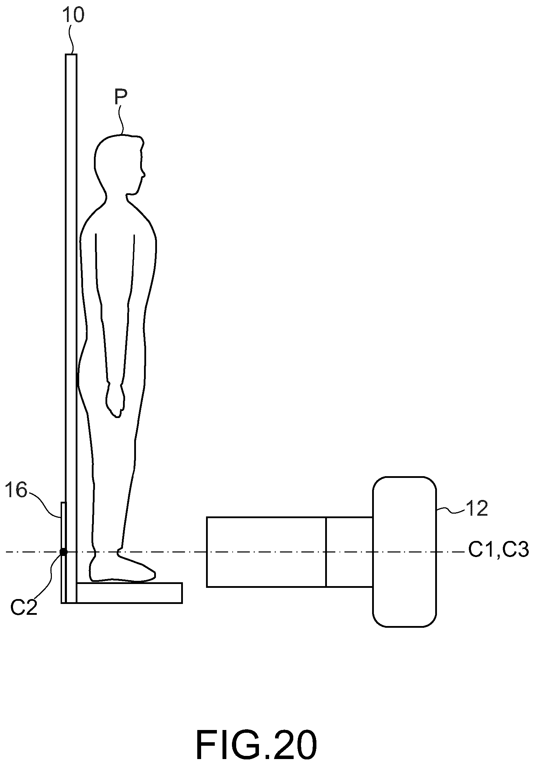

Note that, in the embodiments described above, the position of X-ray focus FC may be set based on an inspection information about an inspection performed on the subject P. Here, inspection information refers to information about inspection performed on the subject P, which at least includes information about an inspection target of the subject P. For this reason, the X-ray diagnosis apparatus 1 may be configured such that the position of X-ray focus FC is set to the front of the inspection target of the subject P based on the information about the inspection target. Specifically, for instance, when "ankle" is included in the inspection information as information about the inspection target, the X-ray diagnosis apparatus 1 may control the X-ray tube holding apparatus 12 to set the position of X-ray focus FC to the front of the ankle of subject P as shown in Fig. 20. As such, by the position of X-ray focus FC being set based on the inspection information, an image suitable for interpretation may be obtained since the image of the inspection target of subject P is the image with small distortion.

Note that, in each of the embodiments described above, the position of X-ray focus FC may receive the input operation about the position of X-ray focus FC and be set based on the received input operation. Specifically, the X-ray diagnosis apparatus may receive the input operation that designates an observation target of subject P, for instance, based on clinical purposes, and set the position of X-ray focus FC to be in the front of the observation target of subject P that is designated, in response to the received input operation. Specifically, for instance, when the X-ray diagnosis apparatus 1 receives the input operation that designates "ankle" via the input circuit 22 as the observation target of subject P, the X-ray diagnosis apparatus 1 may control the X-ray tube holding apparatus 12 such that the position of X-ray focus FC is set to the front of the ankle of subject P. Even in this case, the image suitable for interpretation may be obtained since the image of the observation target of subject P that is designated is the image with small distortion.

Note that, in each of the embodiments described above, the configuration and operation of the X-ray diagnosis apparatus 1 was described with and example where long range imaging was performed with subject P standing, but for the X-ray diagnosis apparatus 1 according to each embodiments described above, the subject P is not limited to be standing, and long range imaging may be realized for arbitrary positions. For instance, the X-ray diagnosis apparatus 1 may perform long range imaging for all or part of the subject where the subject is face-up on the bed 10.

When the subject P is face-up on the bed 10, terms such as upper end, upper end portion, and upper side etc., in each embodiment described above refers to a direction where the head of the subject P is located, and terms such as lower end, lower end portion, and lower side etc., refers to a direction of the feet of the subject. These terms are just provided for convenience in describing the X-ray diagnosis apparatus 1 according to each embodiment. For instance, the terms upper end, upper end portion, and upper side may each be replaced by right end, right end portion, and right side, respectively, and the terms lower end, lower end portion, and lower side may each be replaced by left end, left end portion, and left side. In other words, these terms may be said to point to two opposite directions around some part of the subject P.

While certain embodiments have been described, these embodiments have been presented by way of example only and are not intended to limit the scope of the inventions. The embodiments may be in a variety of other forms. Furthermore, various omissions, substitutions and changes may be made without departing from the scope of the inventions as defined by the appended claims.

## Claims

1. An X-ray diagnosis apparatus comprising:
an X-ray image acquisition unit (18a) configured to acquire an X-ray image by performing an X-ray imaging using an X-ray tube (12a) which irradiates X-rays to a subject (P) and an X-ray detector (16) which detects the X-rays irradiated by the X-ray tube (12a); and
an imaging control unit (18b) configured to control the X-ray tube (12a) and the X-ray detector (16) so that each X-ray focus (FC) in a plurality of shots of the X-ray imaging is positioned at an equivalent position by controlling a focus-to-detector distance, a position of the X-ray detector (16), and an angle of irradiating X-rays to the subject, the focus-to-detector distance being a distance from the X-ray focus (FC) of the X-ray tube (12a) to the X-ray detector (16),
wherein for each X-ray focus (FC), the equivalent position is a position such that artifacts arising from differences in table to object distance are suppressed.

2. The X-ray diagnosis apparatus of Claim 1, wherein the imaging control unit (18a) adjusts a range of irradiating X-rays on a subject by controlling an X-ray aperture (12b) that narrows the range where the X-ray tube irradiates X-ray to narrow a region of an end portion when X-ray imaging the X-ray image of an upper end or a lower end in the plurality of shots of X-ray imaging.

3. The X-ray diagnosis apparatus of Claim 2, wherein a position of the X-ray focus (FC) and the angle of irradiating X-rays to the subject are decided in a fixed manner based on a number of shots of the X-ray imaging.

4. The X-ray diagnosis apparatus of one of Claims 1 to 3, wherein the imaging control unit (18b) adjusts the focus-to-detector distance in response to the angle of irradiating X-rays to the subject (P) such that each X-ray focus (FC) of the X-ray tube (12a) is positioned at the equivalent position in the plurality of shots of X-ray imaging.

5. The X-ray diagnosis apparatus of one of Claims 1 to 2, wherein the imaging control unit (18b) adjusts a range of irradiating X-rays on a subject (P) by controlling an X-ray aperture (12b) that narrows the range where the X-ray tube irradiates X-ray to narrow both regions of an upper end portion and a lower end portion when X-ray imaging the X-ray image of an upper end or a lower end in the plurality of shots of X-ray imaging.

6. The X-ray diagnosis apparatus of one of Claims 1 to 2, wherein the imaging control unit (18b) adjusts a range of irradiating X-rays on a subject (P) by controlling an X-ray aperture (12b) that narrows the range where the X-ray tube irradiates X-ray to narrow a region of an end portion when X-ray imaging the X-ray image of at least one of an upper end or a lower end in the plurality of shots of X-ray imaging.

7. The X-ray diagnosis apparatus of one of Claims 1 to 2, wherein the imaging control unit (18b) adjusts a range of irradiating X-rays on a subject (P) by controlling an X-ray aperture that narrows the range where the X-ray tube irradiates X-ray in response to a position of an upper end of long range imaging and a number of shots of X-ray imaging, to narrow an upper end portion for a first shot of X-ray imaging when X-ray imaging the X-ray image of an upper end in the plurality of shots of X-ray imaging.

8. The X-ray diagnosis apparatus of one of Claims 1 to 2, wherein a position of the X-ray focus (FC) is set to a center portion of the range to image the subject (P) by the plurality of shots of X-ray imaging.

9. The X-ray diagnosis apparatus of one of Claims 1 to 2, wherein a position of the X-ray focus (FC) is set based on an inspection information about an inspection performed on the subject (P).

10. The X-ray diagnosis apparatus of one of Claims 1 to 2, wherein a position of the X-ray focus (FC) is set in response to an input operation received by an input operation from an operator about setting the position of the X-ray focus (FC).

11. The X-ray diagnosis apparatus of one of Claims 1 to 10, further comprising an image generation unit (18c) that generates a long range image of a subject (P) by composing the plurality of X-ray images acquired by the plurality of shots of X-ray imaging.

12. The X-ray diagnosis apparatus of one of Claims 1 to 11, wherein the imaging control unit (18b) controls the X-ray tube (12a) such that the angle of irradiating X-rays to the subject (P) rotates around a center of rotation located on a central axis of the X-ray detector (16).

13. The X-ray diagnosis apparatus of one of Claims 1 to 12, wherein the imaging control unit (18b) controls the X-ray tube (12a) such that the angle of irradiating X-rays to the subject (P) rotates around the position of the X-ray focus of the X-ray tube (12a).

14. A method of controlling an X-ray detector, comprising:
a step of acquiring an X-ray image by performing an X-ray imaging using an X-ray tube (12a) which irradiates X-rays to a subject (P) and an X-ray detector (16) which detects the X-rays irradiated by the X-ray tube; and
a step of controlling the X-ray tube (12a) and the X-ray detector (16) so that each X-ray focus (FC) in a plurality of shots of the X-ray imaging is positioned at an equivalent position by controlling a focus-to-detector distance, a position of the X-ray detector (16), and an angle of irradiating X-rays to the subject (P), the focus-to-detector distance being a distance from the X-ray focus (FC) of the X-ray tube (12a) to the X-ray detector (16),
wherein for each X-ray focus (FC), the equivalent position is a position such that artifacts arising from differences in table to object distance are suppressed.

## Patentansprüche

1. Röntgendiagnosegerät, Folgendes umfassend:
eine Röntgenbild-Erfassungseinheit (18a), die so konfiguriert ist, dass sie ein Röntgenbild durch Durchführen einer Röntgenbildgebung unter Verwendung einer Röntgenröhre (12a), die Röntgenstrahlen auf einen Probanden (P) strahlt, und eines Röntgendetektors (16), der die von der Röntgenröhre (12a) ausgestrahlten Röntgenstrahlen erkennt, erfasst; und
eine Bildgebungssteuereinheit (18b), die so konfiguriert ist, dass sie die Röntgenröhre (12a) und den Röntgendetektor (16) so steuert, dass jeder Röntgenfokus (FC) in einer Vielzahl von Aufnahmen der Röntgenbildgebung an einer äquivalenten Position positioniert wird, indem ein Fokus-zu-Detektor-Abstand, eine Position des Röntgendetektors (16) und ein Winkel der auf den Probanden einstrahlenden Röntgenstrahlen gesteuert werden, wobei der Fokus-zu-Detektor-Abstand ein Abstand vom Röntgenfokus (FC) der Röntgenröhre (12a) zum Röntgendetektor (16) ist,
wobei für jeden Röntgenfokus (FC) die äquivalente Position eine derartige Position ist, dass Artefakte, die durch Differenzen im Abstand zwischen Tisch und Objekt entstehen, unterdrückt werden.

2. Röntgendiagnosegerät nach Anspruch 1, wobei die Bildgebungssteuereinheit (18a) einen Bereich von auf einen Probanden einstrahlenden Röntgenstrahlen durch Steuern einer Röntgenblende (12b) einstellt, die den Bereich verengt, in dem die Röntgenröhre Röntgenstrahlen einstrahlt, um einen Bereich eines Endabschnitts beim Röntgenbildgeben des Röntgenbildes eines oberen Endes oder eines unteren Endes in der Vielzahl von Röntgenbildgebungs-Aufnahmen zu verengen.

3. Röntgendiagnosegerät nach Anspruch 2, wobei eine Position des Röntgenfokus (FC) und der Winkel der auf den Probanden einstrahlenden Röntgenstrahlen auf eine feste Weise basierend auf einer Anzahl von Röntgenbildgebungs-Aufnahmen beschlossen werden.

4. Röntgendiagnosegerät nach einem der Ansprüche 1 bis 3, wobei die Bildgebungssteuereinheit (18b) den Fokus-zu-Detektor-Abstand als Reaktion auf den Winkel der auf den Probanden (P) einstrahlenden Röntgenstrahlen so einstellt, dass jeder Röntgenfokus (FC) der Röntgenröhre (12a) an der äquivalenten Position in der Vielzahl von Röntgenbildgebungs-Aufnahmen positioniert wird.

5. Röntgendiagnosegerät nach einem der Ansprüche 1 bis 4, wobei die Bildgebungssteuereinheit (18b) einen Bereich von Röntgenstrahlen, die auf einen Probanden (P) einstrahlen, durch Steuerung einer Röntgenblende (12b) einstellt, die den Bereich verengt, in dem die Röntgenröhre Röntgenstrahlen abstrahlt, um beide Bereiche eines oberen Endabschnitts und eines unteren Endabschnitts beim Röntgenbildgeben des Röntgenbildes eines oberen Endes oder eines unteren Endes in der Vielzahl von Röntgenbildgebungs-Aufnahmen zu verengen.

6. Röntgendiagnosegerät nach einem der Ansprüche 1 bis 5, wobei die Bildgebungssteuereinheit (18b) einen Bereich von Röntgenstrahlen, die auf einen Probanden (P) einstrahlen, durch Steuerung einer Röntgenblende (12b) einstellt, die den Bereich verengt, in dem die Röntgenröhre Röntgenstrahlen abstrahlt, um einen Abschnitt eines Endabschnitts beim Röntgenbildgeben des Röntgenbildes mindestens eines oberen Endes oder eines unteren Endes in der Vielzahl von Röntgenbildgebungs-Aufnahmen zu verengen.

7. Röntgendiagnosegerät nach einem der Ansprüche 1 bis 6, wobei die Bildgebungssteuereinheit (18b) einen Bereich von Röntgenstrahlen, die auf einen Probanden (P) einstrahlen, durch Steuerung einer Röntgenblende einstellt, die den Bereich verengt, in dem die Röntgenröhre Röntgenstrahlen abstrahlt, als Reaktion auf eine Position eines oberen Endes einer Fernbereichsbildgebung und eine Anzahl von Röntgenbildgebungs-Aufnahmen, um einen oberen Endabschnitt für eine erste Röntgenbildgebungs-Aufnahme beim Röntgenbildgeben des Röntgenbildes eines oberen Endes in der Vielzahl von Röntgenbildgebungs-Aufnahmen zu verengen.

8. Röntgendiagnosegerät nach einem der Ansprüche 1 bis 7, wobei eine Position des Röntgenfokus (FC) auf einen mittleren Abschnitt des Bereichs eingestellt ist, um den Probanden (P) durch die Vielzahl von Röntgenbildgebungs-Aufnahmen abzubilden.

9. Röntgendiagnosegerät nach einem der Ansprüche 1 bis 8, wobei eine Position des Röntgenfokus (FC) auf der Grundlage von Untersuchungsinformationen über eine am Probanden (P) durchgeführte Untersuchung eingestellt wird.

10. Röntgendiagnosegerät nach einem der Ansprüche 1 bis 9, wobei eine Position des Röntgenfokus (FC) als Reaktion auf eine Eingabeoperation eingestellt wird, die durch eine Eingabeoperation von einem Bediener zum Einstellen der Position des Röntgenfokus (FC) empfangen wird.

11. Röntgendiagnosegerät nach einem der Ansprüche 1 bis 10, ferner umfassend eine Bilderzeugungseinheit (18c), die ein Fernbereichsbild eines Probanden (P) durch Zusammensetzen der Vielzahl von Röntgenbildern erzeugt, die durch die Vielzahl von Röntgenbildgebungs-Aufnahmen erfasst werden.

12. Röntgendiagnosegerät nach einem der Ansprüche 1 bis 11, wobei die Bildgebungssteuereinheit (18b) die Röntgenröhre (12a) so steuert, dass der Winkel der auf den Probanden (P) einstrahlenden Röntgenstrahlen um einen Rotationsmittelpunk rotiert, der sich auf einer Mittelachse des Röntgendetektors (16) befindet.

13. Röntgendiagnosegerät nach einem der Ansprüche 1 bis 12, wobei die Bildgebungssteuereinheit (18b) die Röntgenröhre (12a) so steuert, dass der Winkel der auf den Probanden (P) einstrahlenden Röntgenstrahlen um die Position des Röntgenfokus der Röntgenröhre (12a) rotiert.

14. Verfahren zur Steuerung eines Röntgendetektors, Folgendes umfassend:
einen Schritt zur Erfassung eines Röntgenbildes durch Durchführen einer Röntgenbildgebung unter Verwendung einer Röntgenröhre (12a), die Röntgenstrahlen auf einen Probanden (P) abstrahlt, und eines Röntgendetektors (16), der die von der Röntgenröhre abgestrahlten Röntgenstrahlen erkennt; und
einen Schritt des Steuerns der Röntgenröhre (12a) und des Röntgendetektors (16) derart, dass jeder Röntgenfokus (FC) in einer Vielzahl von Röntgenbildgebungs-Aufnahmen an einer äquivalenten Position positioniert wird, durch Steuern eines Fokuszu-Detektor-Abstandes, einer Position des Röntgendetektors (16) und eines Winkels der auf den Probanden (P) einstrahlenden Röntgenstrahlen, wobei der Fokus-zu-Detektor-Abstand ein Abstand vom Röntgenfokus (FC) der Röntgenröhre (12a) zum Röntgendetektor (16) ist,
wobei für jeden Röntgenfokus (FC) die äquivalente Position eine derartige Position ist, dass Artefakte, die durch Differenzen im Abstand zwischen Tisch und Objekt entstehen, unterdrückt werden.

## Revendications

1. Appareil de diagnostic à rayons X comprenant :
une unité d'acquisition d'image à rayons X (18a) configurée pour acquérir une image à rayons X en réalisant une imagerie à rayons X à l'aide d'un tube à rayons X (12a) qui irradie des rayons X vers un sujet (P) et un détecteur de rayons X (16) qui détecte les rayons X irradiés par le tube à rayons X (12a) ; et
une unité de commande d'imagerie (18b) configurée pour commander le tube à rayons X (12a) et le détecteur de rayons X (16) de sorte que chaque point focal de rayons X (FC) dans une pluralité de clichés de l'imagerie de rayons X est positionnée au niveau d'une position équivalente en commandant une distance point focal-détecteur, une position du détecteur de rayons X (16) et un angle d'irradiation de rayons X vers le sujet, la distance point focal-détecteur étant une distance du point focal de rayons X (FC) du tube à rayons X (12a) au détecteur de rayons X (16),
dans lequel pour chaque point focal de rayons X (FC), la position équivalente est une position telle que des artefacts émergeant à partir de différences dans la distance table-objet sont supprimés.

2. Appareil de diagnostic à rayons X selon la revendication 1, dans lequel l'unité de commande d'imagerie (18a) ajuste une plage de rayons X d'irradiation sur un sujet en commandant une ouverture de rayons X (12b) qui rétrécit la plage où le tube à rayons X irradie des rayons X pour rétrécir une région d'une portion d'extrémité lors de l'imagerie à rayons X de l'image à rayons X d'une extrémité supérieure ou d'une extrémité inférieure dans la pluralité de clichés d'imagerie à rayons X.

3. Appareil de diagnostic à rayons X selon la revendication 2, dans lequel une position du point focal de rayons X (FC) et l'angle d'irradiation des rayons X vers le sujet sont décidés d'une manière fixe sur la base d'un nombre de clichés de l'imagerie à rayons X.

4. Appareil de diagnostic à rayons X selon l'une des revendications 1 à 3, dans lequel l'unité de commande d'imagerie (18b) ajuste la distance point focal-détecteur en réponse à l'angle d'irradiation de rayons X vers le sujet (P) de telle sorte que chaque point focal de rayons X (FC) du tube à rayons X (12a) est positionnée au niveau de la position équivalente dans la pluralité de clichés d'imagerie à rayons X.

5. Appareil de diagnostic à rayons X selon l'une des revendications 1 à 4, dans lequel l'unité de commande d'imagerie (18b) ajuste une plage d'irradiation de rayons X sur un sujet (P) en commandant une ouverture de rayons X (12b) qui rétrécit la plage où le tube à rayons X irradie des rayons X pour rétrécir les deux régions d'une partie d'extrémité supérieure et d'une partie d'extrémité inférieure lors d'une imagerie à rayons X de l'image à rayons X d'une extrémité supérieure ou d'une extrémité inférieure dans la pluralité de clichés d'imagerie à rayons X.

6. Appareil de diagnostic à rayons X selon l'une des revendications 1 à 5, dans lequel l'unité de commande d'imagerie (18b) ajuste une plage d'irradiation de rayons X sur un sujet (P) en commandant une ouverture de rayons X (12b) qui rétrécit la plage où le tube à rayons X irradie des rayons X pour rétrécir une région d'une partie d'extrémité lors de l'imagerie à rayons X de l'image à rayons X d'au moins une parmi une extrémité supérieure ou une extrémité inférieure dans la pluralité de clichés d'imagerie à rayons X.

7. Appareil de diagnostic à rayons X selon l'une des revendications 1 à 6, dans lequel l'unité de commande d'imagerie (18b) ajuste une plage de rayons X d'irradiation sur un sujet (P) en commandant une ouverture de rayons X qui rétrécit la plage où le tube à rayons X irradie des rayons X en réponse à une position d'une extrémité supérieure d'une imagerie à longue portée et un nombre de clichés d'imagerie à rayons X, pour rétrécir une partie d'extrémité supérieure pour un premier cliché d'imagerie à rayons X lors de l'imagerie à rayons X de l'image à rayons X d'une extrémité supérieure dans la pluralité de clichés d'imagerie à rayons X.

8. Appareil de diagnostic à rayons X selon l'une des revendications 1 à 7, dans lequel une position du point focal de rayons X (FC) est réglée sur une portion centrale de la plage pour réaliser une image du sujet (P) par la pluralité de clichés d'imagerie à rayons X.

9. Appareil de diagnostic à rayons X selon l'une des revendications 1 à 8, dans lequel une position du point focal de rayons X (FC) est réglée sur la base d'informations d'examen concernant un examen réalisé sur le sujet (P).

10. Appareil de diagnostic à rayons X selon l'une des revendications 1 à 9, dans lequel une position du point focal de rayons X (FC) est réglée en réponse à une opération d'entrée reçue par une opération d'entrée provenant d'un opérateur concernant le réglage de la position du point focal de rayons X (FC).

11. Appareil de diagnostic à rayons X selon l'une des revendications 1 à 10, comprenant en outre une unité de génération d'image (18c) qui génère une image à longue portée d'un sujet (P) en composant la pluralité d'images à rayons X acquises par la pluralité de clichés de l'imagerie à rayons X.

12. Appareil de diagnostic à rayons X selon l'une des revendications 1 à 11, dans lequel l'unité de commande d'imagerie (18b) commande le tube à rayons X (12a) de telle sorte que l'angle d'irradiation de rayons X vers le sujet (P) opère une rotation autour d'un centre de rotation situé sur un axe central du détecteur de rayons X (16).

13. Appareil de diagnostic à rayons X selon l'une des revendications 1 à 12, dans lequel l'unité de commande d'imagerie (18b) commande le tube à rayons X (12a) de telle sorte que l'angle d'irradiation de rayons X vers le sujet (P) opère une rotation autour de la position du point focal de rayons X du tube à rayons X (12a).

14. Procédé de commande d'un détecteur de rayons X, comprenant :
une étape consistant à acquérir une image à rayons X en réalisant une imagerie à rayons X à l'aide d'un tube à rayons X (12a) qui irradie des rayons X vers un sujet (P) et d'un détecteur de rayons X (16) qui détecte les rayons X irradiés par le tube à rayons X ; et
une étape consistant à commander le tube à rayons X (12a) et le détecteur de rayons X (16) de sorte que chaque point focal de rayons X (FC) dans une pluralité de clichés de l'imagerie à rayons X est positionné au niveau d'une position équivalente en commandant une distance point focal-détecteur, une position du détecteur de rayons X (16) et un angle d'irradiation de rayons X vers le sujet (P), la distance point focal-détecteur étant une distance du point focal (FC) de rayons X du tube à rayons X (12a) au détecteur de rayons X (16),
dans lequel pour chaque point focal de rayons X (FC), la position équivalente est une position telle que des artefacts émergeant à partir de différences dans une distance table-objet sont supprimés.
